# EUROPEAN PATENT SPECIFICATION

(11) **EP 1 309 701 B1**
(45) Date of publication and mention of the grant of the patent: **05.04.2006**
(21) Application number: 01958217.0
(22) Date of filing: 16.08.2001
(51) Int. Cl.: C12N 15/56, C12N 15/82, C12N 9/24, C07K 14/435, C07K 16/40, C12Q 1/34, C12Q 1/68, A61K 38/00, C07H 1/00, C07H 3/00, A01H 5/00

(54) **MYROSINASE FROM BREVICORYNE BRASSICAE**
MYROSINASE AUS BREVICORYNE BRASSICAE
MYROSINASE A PARTIR DE BREVICORYNE BRASSICAE

(30) Priority: 17.08.2000 GB 0020331
(43) Date of publication of application: 14.05.2003
(73) Proprietor: IMPERIAL COLLEGE OF SCIENCE, TECHNOLOGY & MEDICINE, London SW7 2AZ (GB)
(72) Inventor: ROSSITER, John, Cantebury, Kent CT6 7AJ (GB); BONES, Atle, N-7952 Trondheim (NO); JONES, Alex, Dorset BH12 2EA (GB); WINGE, Per, N-7030 Trondheim (NO)
(74) Representative: Harding, Charles Thomas
(86) International application number: PCT/GB2001/003670
(87) International publication number: WO 2002/016617

(56) References cited:
- EP-A- 0 713 873
- THANGSTAD OLE PETTER ET AL: "The myrosinase (thioglucoside glucohydrolase) gene family in Brassicaceae." PLANT MOLECULAR BIOLOGY, vol. 23, no. 3, 1993, pages 511-524, XP002191631 ISSN: 0167-4412
- BONES A M ET AL.: "FATE OF MYROSIN CELLS CHARACTERIZATION OF MONOCLONAL ANTIBODIES AGAINST MYROSINASE " JOURNAL OF EXPERIMENTAL BOTANY , vol. 42, no. 245, 1991, pages 1541-1550, XP001053719
- SCOFIELD ET AL.: "INHIBITION OF THIOGLUCOSIDASE-CATALYZED GLUCOSINOLATE HYDROLYSIS BY CASTANOSPERMINE AND RELATED ALKALOIDS " PHYTOCHEMISTRY., vol. 29, 1990, pages 107-110, XP001053594 PERGAMON PRESS., GB ISSN: 0031-9422 cited in the application
- LEONI O ET AL: "Myrosinase-Generated Isothiocyanate from Glucosinolates: Isolates, Characterization and In Vitro Antiproliferative Studies" BIOORGANIC & MEDICINAL CHEMISTRY, ELSEVIER SCIENCE LTD, GB, vol. 5, no. 9, 1997, pages 1799-1806, XP002086076 ISSN: 0968-0896 cited in the application
- GRAZIA BOTTI M ET AL: "Studies on the Mechanism of Myrosinase: Investigation of the effect of glycosyl acceptors on enzyme activity." JOURNAL OF BIOLOGICAL CHEMISTRY, vol. 270, no. 35, 1995, pages 20530-20535, XP002190662 ISSN: 0021-9258 cited in the application
- BURMEISTER WIM P ET AL: "The crystal structures of Sinapis alba myrosinase and a covalent glycosyl-enzyme intermediate provide insights into the substrate recognition and active-site machinery of an S-glycosidase." STRUCTURE (LONDON), vol. 5, no. 5, 1997, pages 663-675, XP002191632 ISSN: 0969-2126 cited in the application
- JONES A M E ET AL: "Purification and characterisation of a non-plant myrosinase from the cabbage aphid Brevicoryne brassicae (L.)." INSECT BIOCHEMISTRY AND MOLECULAR BIOLOGY, vol. 31, no. 1, January 2001 (2001-01), pages 1-5, XP002190663 ISSN: 0965-1748

## Description

The present invention relates to an enzyme and a nucleotide sequence encoding such an enzyme.

### BACKGROUND

### Myrosinase

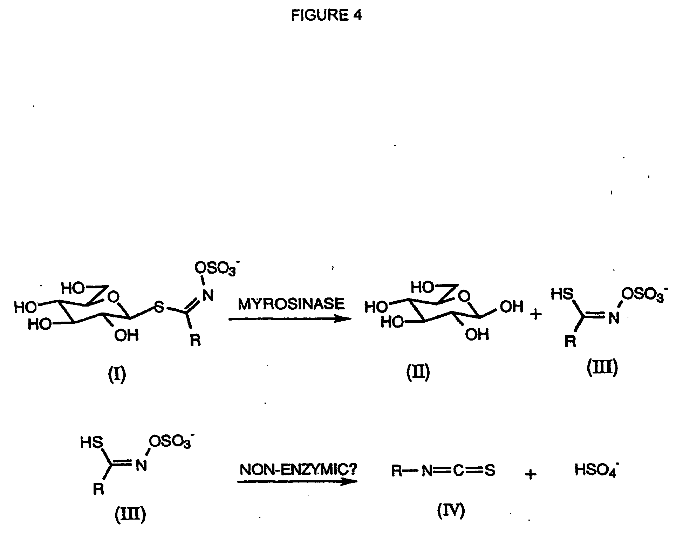

Myrosinase (E.C. number 3.2.3.2, also known as; β-thioglucosidase, β-thioglucoside glucohydrolase) is an enzyme which catalyses the hydrolysis of glucosinolates, a group of naturally occurring sulfur containing glycosides. The metabolism of glucosinolates as catalysed by myrosinase is shown in Figure 4.

Glucosinolates and their degradation products are responsible for the characteristic taste and odour of crops such as horseradish, cabbage, mustard and broccoli (isothiocyanates are responsible for the 'bite' and pungency) and therefore in these crops the glucosinolate content is valued. It is widely accepted that glucosinolates play a role in plant defence against pathogens and insect pests (Bones and Rossiter (1996) Physiologia Plantarum 97:194-208)

The enzyme mediated hydrolysis of glucosinolates leads to a labile aglycone, which rapidly undergoes spontaneous rearrangement, eliminating sulphur, to yield a variety of toxic metabolites such as isothiocyanates, thiocyanates, cyanoepithioalkanes and nitriles. The reaction products depend on pH and other factors such as the presence of ferrous ions, epithiospecifier protein and the nature of the glucosinolate side chain.

The biological importance of glucosinolate metabolism is being increasingly recognised and recent studies have shown that some glucosinolates and/or their breakdown products have pronounced anti-cancer activity (J. W. Fahey, Y. Zhang and P. Talalay, *Proc. Natl. Acad. Sci. USA,* **1997, 94,** 10367-10372).

In particular, isothiocyanates have been shown to be very potent inducers of phase 2 detoxication enzymes (such as glutathione transferases, epoxide hydrolase, NAD(P)H:quinone reductase, and glucuronosyltransferase) which protect against carcinogenesis, mutagenesis and other forms of toxicity of electrophiles and reactive forms, of oxygen (Fahey *et al,* as above).

Glucosinolates make an attractive target for future cancer prevention strategies especially as they are present in a wide range of vegetables such as broccoli, cabbage and Brussels sprouts in reasonably high levels. However, work on establishing the exact mechanism and full biological significance of the anti-cancer effects is being hampered by a poor understanding of the metabolism of glucosinolates.

In order to better understand the metabolism of glucosinolates, it would be desirable to be able to obtain large quantities of myrosinase for further experimentation. Unfortunately, previous attempts to produce functional recombinant plant myrosinase in *E. coli* have met with limited success (S.Chen and B.Halkier, 1999, Protein Expression and Purification, Vol 17 (3) p421.

### Plant myrosinase

Plant myrosinase is very specific for the glucosinolate structure, although there is little discrimination between glucosinolates particularly with similar types of side chain. It has been demonstrated that the sulfate group is absolutely required for activity and the desulfoglucosinolate has been found to be a competitive inhibitor for the enzyme (Ettlinger *et al* Proc. Natl. Acad. Sci. 1961 **47**:1875; Hanley *et al* J. Sci Food Agric 1990 **51**:417) The only O-β-glycosides which are hydrolysed by the enzyme are *p*-nitrophenyl-β-glucoside and *o*-nitrophenyl-β-glucoside. A number of S-β-glucosides have been examined and none of them found to be substrates for the enzyme. In contrast to most β-glucosidases it has also been shown that plant myrosinase does not catalyse transglycosylation reactions where the glycosyl fragment is trapped out by alternative acceptors to water (Botti *et al* 1995 J. Biol. Chem. **270**:20530-20535) Potent inhibitors of β-glucosidases, such as D-glucono-β-lactone, are poor inhibitors of plant myrosinase. Plant myrosinases are activated by low concentrations of ascorbic acid, although they are inhibited by high concentrations.

Myrosinase has been isolated and purified from a range of plant sources. Recently the native enzyme from white mustard seed (*Sinapis alba*) has been crystallised and the X-ray structure determined to 1.6 Å (W. P. Burmeister, S. Cottaz, H. Driguez, R. lori, S. Palmieri and B. Henrissat, *Structure,* 1997, **5**, 663-675). The enzyme was found to closely resemble the cyanogenic β-glucosidase from white clover, folding into a (α/β)₈₋barrel structure. 2-Deoxy-2-fluoroglucotropaeolin (Cottaz *et al* (1996) Biochemistry **35**;15256-15259) was found to be a potent inhibitor of the enzyme, due to the formation of a stable glycosyl-enzyme intermediate at the active site.

A major difference was observed in the catalytic machinery at the active site between plant myrosinase and other glycosidases. Most β-glucosidases have two catalytically important glutamic acid residues at the active site. One of these is the catalytic nucleophile which forms a covalent bond with C-1 of the glycosyl unit. The other acts as an acid catalyst and protonates the aglycone assisting its departure. In myrosinase Glu409 acts as the catalytic nucleophile, but the second glutamic acid is absent and is replaced by a glutamine residue (Gln187). The lack of the second glutamic acid can be rationalised because the glucosinolate aglycone is a very good leaving group, with an estimated pKa of 3.0, and so does not require protonation. This also helps explain two other observations. Firstly, the *p*-nitrophenyt-β-giucosides are the only *O*-glycoside substrates turned over because they are the only compounds tested with leaving groups that are sufficiently acidic to not require protonation. Secondly the absence of a transglycosylation activity probably results from the lack of a glutamate residue to act as a base in the reverse reaction to deprotonate the glycosyl acceptor. This fits with the observation that the only glycosyl acceptor to show any activity at all was azide, which is already anionic (Botti et al (1995) as above).

With respect to binding of the glucosinolate substrate, a hydrophobic pocket was observed, which was ideally situated to bind the hydrophobic side chain of the glucosinolate. Docking of a substrate, sinigrin, into the structure was carried out to identify the binding interactions. The crystal structure of sinigrin could not be used directly for the modelling as this produced clashes between the enzyme and the glucose ring. However, work by Sulzenbacher *et al.* had shown that for a retaining cellulase catalysis takes place via a twist boat conformation, with a quasi-axial orientation for the leaving group (Sulzenbacher *et al.* 1996 Biochemistry 35 15280-15287). A similar conformation for sinigrin gave a much improved fit in the active site of myrosinase. This positioned two arginine residues (Arg194 and Arg259) such that they should be able to interact with the sulfate group. These two residues are strictly conserved among plant myrosinases but are absent in the related β-glucosidases. The glutamine (Gln187), which is also conserved in all known myrosinase sequences, can also hydrogen bond to the sulfate group. A glutamic acid residue at this position would cause unfavourable interactions with the sulfate group.

### Myrosinase from other sources

There are a number of reports of myrosinase-like activity from various other sources including fungi, (M. Ohtsuru, I. Tsuruo and T. Hata, *Agric. Biol. Chem.,* 1969, **33,** 1315-1319; 1320-1325; and M. Ohtsuru and T. Hata, *Agric. Biol. Chem.,* 1973, **37,** 2543-2548), intestinal bacteria (N. Tani, M. Uhtsuru and T. Hata, *Agric. Biol. Chem.,* 1974, **38**, 1617-1622; E. L. Oginsky, A. E. Stein and M. A. Greer, *Proc. Soc. Exp. Med.,* 1965, **119**, 360-364) mammalian tissue (Goodman, J. R. Fouts, E. Bresnick, R. Menegas and G. H. Hitchings, *Science,* 1959, **130,** 450-451) and cruciferous aphids (D. B. MacGibbon and R. M. Allison, *New Zealand J. Sci.,* 1968, **11**, 440-446). Unfortunately many of these previous reports are very sketchy.

The work on bacterial myrosinases has done little more than demonstrate glucosinolate hydrolysis taking place in some bacteria including *Enterobacter cloacae* and *paracolobactrum aerogenoides.* The enzyme was not isolated or characterised in any way. The mammalian metabolism of glucosinolates is still poorly understood. It is generally assumed that the compounds are metabolised by myrosinase activity in the intestinal bacteria, although this has yet to be unambiguously identified. A mammalian myrosinase has been proposed but the putative enzyme has only been shown to hydrolyse thioglycosides and not glucosinolates.

Aphid myrosinase was detected several decades ago in *Brevicoryne brassicae* L (cabbage aphid) (D. B. MacGibbon and R. M. Allison, *New Zealand J. Sci.,* 1978, **21**, 389-392) and in *Lipaphis erysimi.* (R. M. Allison, *New Zealand J. Sci.,* 1968, **11**, 440-446). These myrosinases, when extracted from aphid tissue, were capable of hydrolysing the glucosinolate progoitrin and on examination by electrophoresis appeared to be different from the enzymes found in their cruciferous host plants. The origin of the myrosinase was unclear and was considered by some to be modified plant myrosinase or possibly from gut symbionts.

Despite various reports of myrosinase activity in non-plant sources, the isolation of myrosinase from a non-plant source has not been reported to date.

### The role of myrosinases in plants

In plants the enzymic hydrolysis of glucosinolates by myrosinase usually occurs when cells are damaged, as a result of plant injury or food processing. The hydrolysis products are β-D-glucose and the aglycone fragment. The aglycone produced is unstable and reacts further to give the isothiocyanate by means of a Lossen type rearrangement. Other products may also be produced from the aglycone depending on the reaction conditions, including thiocyanates, nitriles, amines and oxazolidine-2-thiones.

Plant myrosinases are activated by low concentrations of ascorbic acid, although they are inhibited by high concentrations.

Plant myrosinases and glucosinolates constitute a defence system in cruciferous plants towards pests and diseases. Strategies for boosting myrosinase activity in plants should thus have the effect of increasing the plants protection capacity against pests and diseases.

Also, boosting myrosinase activity in plants should lead to a greater release of isothiocyanates, which may improve the taste and odour of crops such as horseradish, cabbage, mustard and broccoli.

### Anti-aphid insecticides

Aphids are insects of the order Homoptera, often known as plant bugs. These insects have piercing and sucking mouth-parts and feed upon sap. Many species are serious pests of agricultural and horticultural crops and of ornamental plants.

There is considerable interest in methods for minimising or eradicating aphid-associated plant damage and diseases.

Since the late 1940s, methods to control these pests have centred on the exogenous application of synthetic organochemicals. Insecticides of the chlorinated hydrocarbon, substituted phenol, organophosphate, carbamate and pyrethrin classes have been used, but this method of plant protection is encountering increasing problems known to those versed in the art.

For example, various problems are associated with need for exogenous application of the chemicals to the plants. Application is usually achieved by spraying which can be inaccurate, especially if long-range spraying techniques are employed. For example, insecticide sprayed from a plane can be rerouted by wind. Also, exogenously applied chemicals can have limited persistence, since they may be washed off by rain, or be light-sensitive.

There is also the problem of the development of pest insect resistance to pesticides. This phenomenon is particularly acute amongst Homopterans, where the typically short generation time allows the emergence of resistant biotypes very rapidly. For example, the brown planthopper of rice can apparently develop a new biotype in only about 18 months.

Biological control of pest insects has been favoured as an alternative strategy. Such an approach exploits the natural viral, bacterial or fungal pathogens or the natural invertebrate or vertebrate predators of the target pest to limit its population. The widespread introduction of biological control measures has, however, been limited by problems of large scale production, widespread application and lack of persistence of the control agents in the field.

An alternative solution is to use inherently insect resistant cultivars, varieties or lines as part of an integrated pest management programme. Production of such resistant lines, which may exhibit pest avoidance, non-preference, antibiosis or pest tolerance, is a major goal of many conventional plant breeding programmes for crop improvement. The challenge is to find an appropriate source of resistance to a specific pest.

### Sulphated carbohydrates

Sulfated carbohydrates mediate many important extracellular recognition events (K. G. Bowman and C. R. Bertozzi, *Chem.* & *Biol.,* 1999, **6**, R9-R22) These include the Nod factors which are required for root nodulation and infection of legumes (C. Freiberg *et al.*, *Nature,* 1997, **387**, 394-401) and sulfated sialyl Lewis X which is a key modulator of leukocyte-endothelial cell interactions (S. D. Rosen and C. R. Bertozzi, *Curr. Biol.*, 1996, **6**, 261-264).

They have also been suggested in connection with the treatment of various disease conditions. For example, HIV infection (Katsuraya *et al* (1999) Carbohydrate Research **315**, 234-242), HCMV infection (OgawaGoto *et al* (1998) J. Gen Virol. **79**, 2533-2541) and conditions associated with irregularities in blood clotting (Akashi *et al* (1996) Bioconjugate Chemistry 7 393-395, Razi and Lindahl (1995) J. Biol. Chem **270** 11267-11275).

Various other important activities have been associated with these carbohydrates, such as: inhibition of specific members of the selectin family (Manning *et al* (1997) Tetrahedron **53**, 11937-11952) and L-selectin-mediated leukocyte rolling (Sanders *et al* (1999) J. Biol. Chem. **274** 5271-5278); macrophage-stimulation activity (Normura *et al* (1998) Bioscience Biotechnology and Biochemistry **62** 1190-1195); binding to platelet-derived growth factors (Feyzi *et al* (1997) J. Biol. Chem. **272** 5518-5524); and triggering the acrosome reaction in marine invertebrates (Hoshi *et al* (1994) Int. J. Developmental Biol. **38,** 167-174).

Sulfated saccharides are very difficult to prepare by chemical means. Hence there is considerable interest in using enzymological methods for synthesis of such glycosides.

### SUMMARY OF THE INVENTION

The present inventors have isolated a myrosinase enzyme from a non-plant source for the first time. The source is an aphid, *Brevicoryne brassicae* L (cabbage aphid), whose main food source is a glucosinolate containing crucifer. The aphid myrosinase has been purified to homogeneity and the sequence of the gene elucidated by RACE-PCR. The availability of a recombinant source of myrosinase protein greatly facilitates investigation into the metabolism or glucosinolates, and the anti-cancer effect of glucosinolates and their breakdown products.

The present inventors have also shown that, unlike plant myrosinase, aphid myrosinase does not require ascorbic acid for activation. Expression of aphid myrosinase within a plant thus provides an alternative strategy for boosting the plant's protection capacity against pests and diseases;

inhibition of the myrosinase *in vivo* (i.e. within an aphid) has an adverse effect on the aphid, making the enzyme an attractive candidate for inhibition by an insecticide.

Also, the present inventors have demonstrated that, unlike plant myrosinase, aphid myrosinase has a critical glutamic acid residue which is required to deprotonate the glycosyl acceptor in a transglycosylation reaction. The capacity of aphid myrosinase to catalyse transglycosylation makes it an excellent candidate biocatalyst for the synthesis of glycosides with charged side chains.

The present inventors also predict that the aphid myrosinase will be capable of catalysing sulphation reactions. There is considerable interest in sulphated carbohydrates and they are notoriously difficult to prepare by chemical means. Aphid myrosinase is therefore also an excellent candidate biocatalyst for the synthesis of sulphated carbohydrates, especially sulphated oligosaccharides.

Thus, according to a first aspect of the invention there is provided a polypeptide, which is a myrosinase enzyme from a non-plant source comprising the amino acid sequence as shown in SEQ ID NO. 1 or a homologue thereof, having at least 75% sequence identity, which displays myrosinase activity and which does not require ascorbic acid for activation.

According to a second aspect of the invention, there is provided a nucleotide sequence capable of encoding such a polypeptide. The nucleotide sequence may comprise the nucleic acid sequence shown in SEQ ID NO: 2 or:
(i) a homologue thereof having at least 75% sequence identity;
(ii) a fragment thereof which has one or more bases missing, but which retains the capacity to encode an enzyme according to claim 1; or
(iii) a derivative thereof which has a chemical modification of the side chains and/or the backbone of the nucleotide sequence but which retains the capacity to encode an enzyme according to claim 1.

In various other aspects, the present invention also provides: an antibody capable of recognising such a polypeptide; a vector comprising such a nucleotide sequence; a host cell and an organism into which has been incorporated such a nucleotide sequence.

In a preferred embodiment, the present invention provides a plant expressing the polypeptide of the first aspect of the invention.

The present invention also provides a method of screening for an agent capable of modulating myrosinase activity and/or expression.

Myrosinases are involved in the hydrolysis of glucosinolates. Glycosinolates and their breakdown products have been demonstrated to have anti-cancer activity. Thus the present invention also provides the use of the polypeptide of the first aspect of the invention, or a nucleotide sequence capable of encoding such a polypeptide, in the manufacture of a medicament for the treatment or prevention of cancer.

The present inventors have shown that, unlike plant myrosinase, aphid myrosinase does not require ascorbic acid for activation. Expression of aphid myrosinase in a plant will therefore enhance the plant's protection capacity against pests and diseases in the absence of ascorbic acid. The present invention also provides a method for enhancing pest and/or disease resistance in a plant which comprises the step of expressing a polypeptide of the first aspect of the invention in the plant.

Plant myrosinases and glucosinolates constitute a defence system in cruciferous plants towards pests and diseases. The present inventors have shown that some specialist insects have evolved a defence system, similar to the plant system, and possess a myrosinase together with sequestered glucosinolates. An inhibitor of the myrosinase characterised by the present inventors should have anti-insect activity. In this respect, inhibition of the myrosinase activtity may block glucosinolate metabolism leading to a build up of glucosinolates, causing toxic effects. Alternatively inhibition of the aphid myrosinase will reduce the release of isothiocyanates such that on tissue damage by a natural predator the combined effect of famesene and isothiocyanate (which constitute an alarm system for other aphids) will be impaired. Inhibition of myrosinase may also make the insect more susceptible to pests and diseases.

The presence of a critical glutamic acidic residue, means that aphid myrosinase is able to catalyse transglycosylation reactions, unlike plant myrosinase. The present invention further provides a method for synthesising a glycoside which comprises the step of using a polypeptide according to the first aspect of the invention to catalyse a transglycosylation reaction.

The present invention further provides a method for synthesising a sulphated carbohydrate which comprises the step of using a polypeptide according to the first aspect of the invention to catalyse a sulphation reaction.

### DETAILED DESCRIPTION OF THE INVENTION

The first aspect of the invention relates to a polypeptide.

### POLYPEPTIDES

The term "polypeptide" - which is interchangeable with the term "protein" - includes single-chain polypeptide molecules as well as multiple-polypeptide complexes where individual constituent polypeptides are linked by covalent or non-covalent means.

Polypeptides of the present invention may be in a substantially isolated form. It will be understood that the polypeptide may be mixed with carriers or diluents which will not interfere with the intended purpose of the polypeptide and still be regarded as substantially isolated. A polypeptide of the present invention may also be in a substantially purified form, in which case it will generally comprise the polypeptide in a preparation in which more than 90%, e.g. 95%, 98% or 99% of the polypeptide in the preparation is a polypeptide of the present invention. Polypeptides of the present invention may be modified for example by the addition of histidine residues to assist their purification or by the addition of a signal sequence to promote their secretion from a cell as discussed below.

Polypeptides of the present invention may be produced by synthetic means (e.g. as described by Geysen *et al.,* 1996). For example, peptides can be synthesized by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure).

Direct peptide synthesis can be performed using various solid-phase techniques (Roberge JY *et al* (1995) Science 269: 202-204) and automated synthesis may be achieved, for example, using the ABI 43 1 A Peptide Synthesizer (Perkin Elmer) in accordance with the instructions provided by the manufacturer. Additionally, the amino acid sequence of myrosinase, or any part thereof, may be altered during direct synthesis and/or combined using chemical methods with a sequence from other subunits, or any part thereof, to produce a variant polypeptide.

The polypeptide may also be produced recombinantly, i.e. by expression of a nucleotide sequence coding for same in a suitable expression system, by known techniques. Myrosinase may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J (1992) Protein Expr Purif 3 -.26328 1), protein A domains that allow purification on immobilised immunoglobulin, and the domain utilised in the FLAGS extension/affinity purification system (Immunex Corp, Seattle, WA). The inclusion of a cleavable linker sequence such as Factor XA or enterokinase (Invitrogen, San Diego, CA) between the purification domain and myrosinase is useful to facilitate purification.

A myrosinase natural, modified or recombinant sequence may be ligated to a heterologous sequence to encode a fusion protein. For example, for screening of peptide libraries for inhibitors of myrosinase activity, it may be useful to encode a chimeric myrosinase protein expressing a heterologous epitope that is recognised by a commercially available antibody. A fusion protein may also be engineered to contain a cleavage site located between a myrosinase sequence and the heterologous protein sequence, so that the myrosinase may be cleaved and purified away from the heterologous moiety.

Preferably, the amino acid sequence *per se* of the present invention does not cover the native myrosinase according to the present invention when it is in its natural environment (i.e. in the cabbage aphid *Brevicoryne brassicacae)* and when it has been expressed by its native nucleotide coding sequence which is also in its natural environment and when that nucleotide sequence is under the control of its native promoter which is also in its natural environment. For ease of reference, we have called this preferred embodiment the "non-native amino acid sequence".

The polypeptide of the first aspect of the present invention comprises the amino acid sequence shown in SEQ ID NO. 1 or a homologue thereof, having at least 75% sequence identity.

### HOMOLOGUE, FRAGMENT AND DERIVATIVE

The term "homologue" covers homology with respect to structure and/or function. With respect to sequence homology, there is at least 75%, more preferably at least 85%, more preferably at least 90% homology to the sequence shown as SEQ ID No. 1 More preferably there is at least 95%, more preferably at least 98%, homology to the sequence shown as SEQ ID No. 1.

The term "fragment" as used herein in relation to the amino acid sequence refers a partial amino acid sequence. The partial amino acid sequence may have one or more amino acids missing from the N-terminal end, the C-terminal end, or the middle of the sequence, but still retain myrosinase function.

The term "derivative" as used herein in relation to the amino acid sequence includes chemical modification of a myrosinase enzyme. Illustrative of such modifications would be replacement of hydrogen by an alkyl, acyl, or amino group.

The terms "homologue", "derivative" or "fragment" in relation to the amino acid sequence for the polypeptide of the present invention include any substitution of, variation of, modification of, replacement of, deletion of or addition of one (or more) amino acid from or to the sequence providing the resultant polypeptide is capable of displaying myrosinase activity, preferably being at least as biologically active as the polypeptide shown in SEQ ID No 1.

Preferably the homologue of the present invention comprises at least 100 contiguous amino acids, preferably at least 200 contiguous amino acids, preferably at least 300 contiguous amino acids, preferably at least 400 contiguous amino acids, preferably at least 430 contiguous amino acids, preferably at least 460 contiguous amino acids, of the amino acid sequence of SEQ ID NO 1.

Typically, for the homologue, derivative or fragment of the present invention, the types of amino acid substitutions that could be made should maintain the hydrophobicity/hydrophilicity of the amino acid sequence. Amino acid substitutions may be made, for example from 1, 2 or 3 to 10, 20 or 30 substitutions provided that the modified sequence retains the ability to act as a myrosinase enzyme in accordance with present invention. Amino acid substitutions may include the use of non-naturally occurring analogues, for example to increase blood plasma half-life.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

As indicated above, proteins of the invention are typically made by recombinant means, for example as described herein, and/or by using synthetic means using techniques well known to skilled persons such as solid phase synthesis. Variants and derivatives of such sequences include fusion proteins, wherein the fusion proteins comprise at least the amino acid sequence of the present invention being linked (directly or indirectly) to another amino acid sequence. These other amino acid sequences - which are sometimes referred to as fusion protein partners - will typically impart a favourable functionality - such as to aid extraction and purification of the amino acid sequence of the present invention. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of the present invention so as to allow removal of the latter. Preferably the fusion protein partner will not hinder the function of the protein of the present invention.

### HOMOLOGY

The term "homology" as used herein may be equated with the term "identity". Here, sequence homology with respect to the amino acid sequence of the present invention can be determined by a simple "eyeball" comparison (i.e. a strict comparison) of any one or more of the sequences with another sequence to see if that other sequence has at least 75% identity to the sequence(s). Relative sequence homology (i.e. sequence identity) can also be determined by commercially available computer programs that can calculate % homology between two or more sequences. A typical example of such a computer program is CLUSTAL

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino adds).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux *et al.,* 1984, Nucleic Acids Research 12:387). Examples of other software than can perform sequence comparisons include, but are not limited to, the BLAST package (see Ausubel *et al.,* 1999 *ibid*- Chapter 18), FASTA (Atschul *et al.,* 1990, J. Mol. Biol., 403-410) and the GENEWORKS suite of comparison tools. Both BLAST and FASTA are available for off-line and on-line searching (see Ausubel *et al.,* 1999 *ibid,* pages 7-58 to 7-60). However, for some applications it is preferred to use the GCG Bestfit program.

Although the final % homology can be measured in terms of identity, in some cases, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default value for the GCG packages, or in the case of other software, the default matrix, such as BLOSUM62.

Once the software has produced an optimal alignment, it is possible to calculate % homology, preferably % sequence identity. The software typically does this as part of the sequence comparison and generates a numerical result.

As indicated, for some applications, sequence homology (or identity) may be determined using any suitable homology algorithm, using for example default parameters. For a discussion of basic issues in similarity searching of sequence databases, see Altschul et al (1994) Nature Genetics 6:119-129. For some applications, the BLAST algorithm is employed, with parameters set to default values. The BLAST algorithm is described in detail at http://www.ncbi.nih.gov/BLAST/blast_help.html. Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

Should Gap Penalties be used when determining sequence identity, then preferably the following parameters are used:

| | |
|---|---|
| **FOR BLAST** | |
| GAP OPEN | 0 |
| GAP EXTENSION | 0 |

| **FOR CLUSTAL** | DNA | PROTEIN | |
|---|---|---|---|
| WORD SIZE | 2 | 1 | K triple |
| GAP PENALTY | 10 | 10 | |
| GAP EXTENSION | 0.1 | 0.1 | |

Other computer program methods to determine identify and similarity between the two sequences include but are not limited to the GCG program package (Devereux et al 1984 Nucleic Acids Research 12: 387 and FASTA (Atschul et al 1990 J Molec Biol 403-410).

### ASSAYS FOR MYROSINASE ACTIVITY

The polypeptide of the first aspect of the invention is capable of displaying myrosinase activity. The term "myrosinase activity" is intended to refer to any activity which is characteristic of aphid myrosinase, such as the capacity to hydrolyse glucosinolates, or the capacity to catalyse transglycosylation and/or sulphation reactions. Myrosinase activity can be measured *in vivo* or *in vitro* using methods known in the art. For example, the capacity of myrosinase to hydrolyse the glycosinolate progoitrin can be measured as described in MacGibbon and Allison 1968 and 1978 (as above). Myrosinase activity can also be measured based on the determination of glucose released by the hydrolysis of 2-propenyl glucosinolate (sinigrin) as described in the Examples.

Preferably the polypeptide of the invention is capable of displaying at least 50%, more preferably at least 75%, most preferably at least 95% of the myrosinase activity displayed by a polypeptide having the amino acid sequence shown in SEQ ID NO. 1.

In a second aspect aspect, the present invention provides a nucleotide sequence capable of encoding a polypeptide of the first aspect of the invention.

### POLYNUCLEOTIDE

The term "nucleotide sequence" as used herein refers to an oligonucleotide sequence or polynucleotide sequence. The nucleotide sequence may be DNA (including cDNA) or RNA which may be of genomic or synthetic or recombinant origin which may be double-stranded or single-stranded whether representing the sense or antisense strand.

In a preferred embodiment, the nucleotide sequence *per se* of the present invention does not cover the native nucleotide coding sequence according to the present invention in its natural environment when it is under the control of its native promoter which is also in its natural environment. For ease of reference, we have called this preferred embodiment the "non-native nucleotide sequence".

The nucleotide sequences of the present invention may include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the nucleotide sequences described herein may be modified by any method available in the art. Such modifications may be carried out in to enhance the *in vivo* activity or life span of nucleotide sequences of the present invention.

The present invention also encompasses nucleotide sequences that are complementary to the sequences presented herein. If the sequence is complementary to a fragment thereof then that sequence can be used a probe to identify similar coding sequences in other organisms etc.

The present invention also encompasses nudeotide sequences that are capable of hybridising to the sequences presented herein. Preferably they are capable of hydridising under conditions of intermediate to maximal stringency. For example, stringent conditions may be 65°C and 0.1xSSC {1×SSC = 0.15 M NaCl, 0.015 Na₃ citrate pH 7.0}.

Polynucleotides such as a DNA polynucleotide and primers according to the present invention may be produced recombinantly, synthetically, or by any means available to those of skill in the art. They may also be cloned by standard techniques.

The nucleotide sequence of the present invention may comprise the nucleic acid sequence shown in SEQ ID No. 2 or
(i) a homologue thereof having at least 75% sequence identity;
(ii) a fragment thereof which has one or more bases missing, but which retains the capacity to encode an enzyme according to claim 1; or
(iii) a derivative thereof which has a chemical modification of the side chains and/or the backbone of the nudeotide sequence but which retains the capacity to encode an enzyme according to claim 1.

### HOMOLOGUE, FRAGMENT AND DERIVATIVE

The term "homologue" covers homology with resped to structure and/or function providing the resultant nucleotide sequence codes for or is capable of coding for an enzyme having myrosinase activity. With respect to sequence homology, there is at least 75%, more preferably at least 85%, more preferably at least 90% homology to a nucleotide sequence coding for the amino acid sequence shown as SEQ ID No. 1. More preferably there is at least 95%, more preferably at least 98% homology to a nucleotide sequence coding for the amino acid sequence shown as SEQ ID No. 1. Preferably, with respect to sequence homology, preferably there is at least 75%, more preferably at least 85%, more preferably at least 90% homology to the sequence shown as SEQ ID No. 2. More preferably there is at least 95%, more preferably at least 98%, homology to the sequence shown as SEQ ID No. 2.

The degree of homology between two nucleic acid sequences can be measured using methods known in the art, as described in the "homology" section above. For some applications, a preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

The term also encompasses sequences that are complementary to sequences that are capable of hydridising to the nucleotide sequences presented herein.

The term "fragment" as used herein in relation to the nucleotide sequence refers to a partial nucleic acid sequence. The partial nucleic acid sequence may have one or more bases missing from the 5'- end, the 3'- end, or the middle of the sequence, but still retain the capacity to encode a polypeptide having myrosinase function.

The term "derivative" as used herein in relation to the nucleotide sequence includes chemical modification of the side chains and/or the backbone of the nucleotide sequence. Such modifications are often made to enhance the solubility, efficacy and/or half life of a nucleotide sequence.

The terms "homologue", "derivative" or "fragment" in relation to the nucleotide sequence coding for the preferred enzyme of the present invention include any substitution of, variation of, modification of, replacement of, deletion of or addition of one (or more) nucleic acid from or to the sequence, providing the resultant nucleotide sequence codes for or is capable of coding for an enzyme having myrosinase activity.

The present invention also provides a vector comprising the nucleotide sequence of the second aspect of the invention.

### VECTORS

The term ''vector'' includes expression vectors and transformation vectors and shuttle vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *E.coli* plasmid to a bacterium, such as of the genus Bacillus, then the transformation vector is sometimes called a "shuttle vector". It may even be a construct capable of being transferred from an *E.coli* plasmid to an Agrobacterium to a plant.

The vectors of the present invention may be transformed into a suitable host cell as described below to provide for expression of a polypeptide of the present invention. Thus, in a further aspect the invention provides a process for preparing polypeptides according to the present invention which comprises cultivating a host cell transformed or transfected with an expression vector as described above under conditions to provide for expression by the vector of a coding sequence encoding the polypeptides, and recovering the expressed polypeptides.

The vectors may be for example, plasmid, virus or phage vectors provided with an origin of replication, optionally a promoter for the expression of the said polynucleotide and optionally a regulator of the promoter.

The vectors of the present invention may contain one or more selectable marker genes. The most suitable selection systems for industrial micro-organisms are those formed by the group of selection markers which do not require a mutation in the host organism. Examples of fungal selection markers are the genes for acetamidase (amdS), ATP synthetase, subunit 9 (*oli*C), orotidine-5'-phosphate-decarboxylase (*pvr*A), phleomycin and benomyl resistance (benA). Examples of non-fungal selection markers are the bacterial G418 resistance gene (this may also be used in yeast, but not in filamentous fungi), the ampicillin resistance gene (*E. coli*), the neomycin resistance gene *(Bacillus)* and the *E.coli uid*A gene, coding for β-glucuronidase (GUS).

Vectors may be used *in vitro,* for example for the production of RNA or used to transfect or transform a host cell.

Thus, polynucleotides of the present invention can be incorporated into a recombinant vector (typically a replicable vector), for example a cloning or expression vector. The vector may be used to replicate the nucleic acid in a compatible host cell. Thus in a further embodiment, the invention provides a method of making polynucleotides of the present invention by introducing a polynucleotide of the present invention into a replicable vector, introducing the vector into a compatible host cell, and growing the host cell under conditions which bring about replication of the vector. The vector may be recovered from the host cell. Suitable host cells are described below in connection with expression vectors.

In a preferred embodiment, the vector is suitable for expression in a yeast system (such as Pichia) or a baculovirus-insect cell system. Methods for expression of the nucleotide in these systems are given in the Examples.

The present invention also provides a host cell into which has been incorporated the nucleotide sequence of the second aspect of the invention.

### HOST CELLS

The term "host cell" - in relation to the present invention includes any cell that could comprise the nucleotide sequence coding for the recombinant protein according to the present invention and/or products obtained therefrom, wherein a promoter can allow expression of the nucleotide sequence according to the present invention when present in the host cell.

Thus, a further embodiment of the present invention provides host cells transformed or transfected with a polynucleotide of the present invention. Preferably said polynucleotide is carried in a vector for the replication and expression of said polynucleotides. The cells will be chosen to be compatible with the said vector and may for example be prokaryotic (for example bacterial), fungal, yeast or plant cells.

The gram-negative bacterium *E. coli* is widely used as a host for heterologous gene expression. However, large amounts of heterologous protein tend to accumulate inside the cell. Subsequent purification of the desired protein from the bulk of *E.coli* intracellular proteins can sometimes be difficult.

In contrast to *E.coli,* bacteria from the genus *Bacillus* are very suitable as heterologous hosts because of their capability to secrete proteins into the culture medium. Other bacteria suitable as hosts are those from the genera *Streptomyces* and *Pseudomonas.*

Depending on the nature of the polynucleotide encoding the polypeptide of the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Examples of suitable expression hosts within the scope of the present invention are fungi such as *Aspergillus* species (such as those described in EP-A-0184438 and EP-A-0284603) and *Trichoderma* species; bacteria such as *Bacillus* species (such as those described in EP-A-0134048 and EP-A-0253455), *Streptomyces* species and *Pseudomonas* species; and yeasts such as *Kluyveromyces* species (such as those described in EP-A-0096430 and EP-A-0301670) and *Saccharomyces* species. By way of example, typical expression hosts may be selected from *Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis* and *Saccharomyces cerevisiae.*

The use of suitable host cells - such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

In a preferred embodiment a yeast or a baculovirus-insect cell system is used to express the nucleotide sequence.

The present invention also provides an organism into which has been incorporated the nucleotide sequence of the second aspect of the invention.

### ORGANISM

The term "organism" in relation to the present invention includes any organism that could comprise the nucleotide sequence coding for the recombinant protein according to the present invention and/or products obtained therefrom, wherein a promoter can allow expression of the nucleotide sequence according to the present invention when present in the organism. Examples of organisms may include a fungus, yeast or a plant.

The term "transgenic organism" in relation to the present invention includes any organism that comprises the nucleotide sequence coding for the protein according to the present invention and/or products obtained therefrom, wherein the promoter can allow expression of the nucleotide sequence according to the present invention within the organism. Preferably the nucleotide sequence is incorporated in the genome of the organism.

The term "transgenic organism" does not cover the native nucleotide coding sequence according to the present invention in its natural environment when it is under the control of its native promoter which is also in its natural environment. In addition, the present invention does not cover the native protein according to the present invention when it is in its natural environment and when it has been expressed by its native nucleotide coding sequence which is also in its natural environment and when that nucleotide sequence is under the control of its native promoter which is also in its natural environment.

Therefore, the transgenic organism of the present invention includes an organism comprising any one of, or combinations of, the nucleotide sequence coding for the amino acid sequence according to the present invention, plasmids or constructs comprising such a sequence, vectors according to the present invention, and host cells according to the present invention. The transformed cell or organism could prepare acceptable quantities of the desired compound which would be easily retrievable from, the cell or organism.

In a preferred embodiment, the transgenic organism is a plant. In particular, the plant may be a Brassica crop such as (but not limited to) cabbage, oilseed rape, sprouts and broccoli.

### TRANSFORMATION OF HOST CELLS/HOST ORGANISMS

As indicated earlier, the host organism can be a prokaryotic or a eukaryotic organism. Examples of suitable prokaryotic hosts include *E*. *coli, Bacillus subtilis,* yeast *(Pichia)* and Baculovirus-insect cells. Teachings on the transformation of prokaryotic hosts is well documented in the art, for example see Sambrook et al (Molecular Cloning: A Laboratory Manual, 2nd edition, 1989, Cold Spring Harbor Laboratory Press) and Ausubel *et al.,* Current Protocols in Molecular Biology (1995), John Wiley & Sons, Inc.

If a prokaryotic host is used then the nucleotide sequence may need to be suitably modified before transformation - such as by removal of introns.

In another embodiment the transgenic organism can be a yeast. In this regard, yeast have also been widely used as a vehicle for heterologous gene expression. The species *Saccharomyces cerevisiae* has a long history of industrial use, including its use for heterologous gene expression. Expression of heterologous genes in *Saccharomyces cerevisiae* has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 907-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in Saccharomyces cerevisiae and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.). Alternatively a Pichia (methylotrophic yeast) system can be used. Systems using such a host cell are commercially available, such as the "EasySelect" system from Invitrogen.

Several types of yeast vectors are available, including integrative vectors, which require recombination with the host genome for their maintenance, and autonomously replicating plasmid vectors.

In order to prepare the transgenic yeast cells, expression constructs are prepared by inserting the nucleotide sequence of the present invention into a construct designed for expression in yeast Several types of constructs used for heterologous expression have been developed. The constructs contain a promoter active in yeast fused to the nucleotide sequence of the present invention, usually a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells are selected using various selective markers. Among the markers used for transformation are a number of auxotrophic markers such as LEU2, HIS4 and TRP1, and dominant antibiotic resistance markers such as aminoglycoside antibiotic markers, eg G418.

In a preferred embodiment the host organism is a plant. The basic principle in the construction of genetically modified plants is to insert genetic information in the plant genome so as to obtain a stable maintenance of the inserted genetic material.

Several techniques exist for inserting the genetic information; the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

Thus, the present invention also provides a method of transforming a host cell with a nucleotide sequence shown in SEQ ID NO 2 or a derivative, homologue or fragment thereof.

Host cells transformed with a myrosinase nucleotide coding sequence may be cultured under conditions suitable for the expression and recovery of the encoded protein from cell culture. The protein produced by a recombinant cell may be secreted or may be contained intracellularly depending on the sequence and/or the vector used. As will be understood by those of skill in the art, expression vectors containing myrosinase coding sequences can be designed with signal sequences which direct secretion of myrosinase coding sequences through a particular prokaryotic or eukaryotic cell membrane. Other recombinant constructions may join myrosinase coding sequence to nucleotide sequence encoding a polypeptide domain which will facilitate purification of soluble proteins (Kroll *DJ et al* (1993) DNA Cell Biol 12:441-53, see also above discussion of vectors containing fusion proteins).

### ANTIBODIES

The present invention also provides an antibody capable of recognising the polypeptide of the first aspect of the invention.

The amino acid sequence of the present invention can also be used to generate antibodies - such as by use of standard techniques - against the amino acid sequence.

Procedures well known in the art may be used for the production of antibodies to myrosinase polypeptides. Such antibodies include, but are not limited to, polyclonal, monoclonal, chimeric, single chain, Fab fragments and fragments produced by a Fab expression library. Neutralising antibodies, i.e., those which inhibit biological activity of myrosinase polypeptides, are especially preferred for insecticide use (see below).

For the production of antibodies, various hosts including goats, rabbits, rats, mice, etc. may be immunised by injection with the inhibitor or any homologue, fragment or derivative thereof or oligopeptide which retains immunogenic properties. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed.

Monoclonal antibodies to the amino acid sequence may be even prepared using any technique which provides for the production of antibody molecules by continuous cell lines in culture. These include, but are not limited to, the hybridoma technique originally described by Koehler and Milstein (1975 Nature 256:495-497), the human B-cell hybridoma technique (Kosbor *et al* (1983) lmmunol Today 4:72; Cote *et al* (1983) Proc, Natl Acad Sci 80:2026-2030) and the EBV-hybridoma technique (Cole *et al* (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison *et al* (1984) Proc Natl Acad Sci 81:6851-6855; Neuberger *et al* (1984) Nature 312:604-608; Takeda *et al* (1985) Nature 314:452-454). Alternatively, techniques described for the production of single chain antibodies (US-A-4946779) can be adapted to produce inhibitor specific single chain antibodies.

Antibodies may also be produced by inducing *in vivo* production in the lymphocyte population or by screening recombinant immunoglobulin libraries or panels of highly specific binding reagents as disclosed in Orlandi *et al* (1989, Proc Natl Acad Sci 86: 3833-3837), and Winter G and Milstein C (1991; Nature 349:293-299).

Antibody fragments which contain specific binding sites for myrosinase may also be generated. For example, such fragments include, but are not limited to, the F(ab')₂ fragments which can be produced by pepsin digestion of the antibody molecule and the Fab fragments which can be generated by reducing the disulphide bridges of the F(ab')₂ fragments. Alternatively, Fab expression libraries may be constructed to allow rapid and easy identification of monoclonal Fab fragments with the desired specificity (Huse WD *et al* (1989) Science 256:1275-1281).

An alternative technique involves screening phage display libraries where, for example the phage express scfv fragments on the surface of their coat with a large variety of complementarity determining regions (CDRs). This technique is well known in the art.

Myrosinase-specific antibodies are useful to test for expression of a related myrosinase enzyme in other organisms (such as other insects), and to examine the distibution of expression within tissues. A variety of protocols for competitive binding or immunoradiometric assays using either polyclonal or monoclonal antibodies with established specificities are well known in the art. Such immunoassays typically involve the formation of complexes between myrosinase polypeptides and its specific antibody (or similar myrosinase -binding molecule) and the measurement of complex formation. A two-site, monoclonal based immunoassay utilising monoclonal antibodies reactive to two non-interfering epitopes on a specific myrosinase protein is preferred, but a competitive binding assay may also be employed. These assays are described in Maddox DE *et al* (1983, J Exp Med 158:121 1).

### SCREENING METHODS

The present invention also provides a method for screening for an agent capable of modulating myrosinase activity and/or expression, and an agent identified by such a screening method.

The screening method of the present invention may comprise the following steps:
(i) contacting an agent with a polypeptide according to the first aspect of the invention or a nucleic acid according to the second aspect of the invention;
(ii) measuring the activity and/or expression of myrosinase
wherein a difference between a) myrosinase activity/expression in the absence of agent, and b) myrosinase activity/expression in the presence of agent is indicative that the agent is capable of modulating myrosinase activity and/or expression.

### BINDING STUDIES

In order to find a candidate agent capable of modulating the expression and/or activity of myrosinase, it may be useful to initially carry out a screen for compounds which are capable of binding to myrosinase.

Where the candidate compounds are proteins, in particular antibodies or peptides, libraries of candidate compounds can be screened for binding using phage display techniques. Phage display is a protocol of molecular screening which utilises recombinant bacteriophage. The technology involves transforming bacteriophage with a gene that encodes the library of candidate compounds, such that each phage or phagemid expresses a particular candidate compound. The transformed bacteriophage (which preferably is tethered to a solid support) expresses the appropriate candidate compound and displays it on their phage coat. Specific candidate compounds which are capable of interacting with myrosinase are enriched by selection strategies based on affinity interaction. The successful candidate agents are then characterised. Phage display has advantages over standard affinity ligand screening technologies. The phage surface displays the candidate agent in a three dimensional configuration, more closely resembling its naturally occurring conformation. This allows for more specific and higher affinity binding for screening purposes.

Another method of screening a library of compounds utilises eukaryotic or prokaryotic host cells which are stably transformed with recombinant DNA molecules expressing the library of compounds. Such cells, either in viable or fixed form, can be used for standard binding-partner assays. See also Parce *et al*. (1989) Science 246:243-247; and Owicki *et al.* (1990) Proc. Nat'l Acad. Sci. USA 87;4007-4011, which describe sensitive methods to detect cellular responses. Competitive assays are particularly useful, where the cells expressing the library of compounds are incubated with a labelled antibody known to bind myrosinase, such as ¹²⁵I-antibody, and a test sample such as a candidate compound whose binding affinity to the binding composition is being measured. The bound and free labelled binding partners for myrosinase are then separated to assess the degree of myrosinase binding. The amount of test sample bound is inversely proportional to the amount of labelled anti-myrosinase antibody binding to the myrosinase.

Any one of numerous techniques can be used to separate bound from free binding partners to assess the degree of binding. This separation step could typically involve a procedure such as adhesion to filters followed by washing, adhesion to plastic following by washing, or centrifugation of the cell membranes.

Still another approach is to use solubilized, unpurified or solubilized purified myrosinase either extracted from eukaryotic or prokaryotic host cells. This allows for a "molecular" binding assay with the advantages of increased specificity, the ability to automate, and high drug test throughput.

Another technique for candidate compound screening involves an approach which provides high throughput screening for new compounds having suitable binding affinity, e.g., to myrosinase, and is described in detail in International Patent application no. WO 84/03564 (Commonwealth Serum Labs.), published on September 13 1984. First, large numbers of different small peptide test compounds are synthesised on a solid substrate, e.g., plastic pins or some other appropriate surface; see Fodor *et al.* (1991). Then all the pins are reacted with solubilized myrosinase and washed. The next step involves detecting bound myrosinase. Detection may be accomplished using a monoclonal antibody to myrosinase (a number of which have already been prepared by the inventors using standard procedures). Compounds which interact specifically with myrosinase may thus be identified.

Rational design of candidate compounds likely to be able to interact with myrosinase may be based upon structural studies of the molecular shapes of myrosinase and/or its *in vivo* binding partners. One means for determining which sites interact with specific other proteins is a physical structure determination, e.g., X-ray crystallography or two-dimensional NMR techniques. These will provide guidance as to which amino acid residues form molecular contact regions. For a detailed description of protein structural determination, see, e.g., Blundell and Johnson (1976) Protein Crystallography, Academic Press, New York. In particular, this would provide information on those regions of the myrosinase polypeptide which are involved in homodimerisation, and interaction with pyruvate kinase, hnRNPE1, YP4 and fibrillarin and *vice versa.*

Another technique for drug screening provides for high throughput screening of compounds having suitable binding affinity to the myrosinase polypeptides and is based upon the method described in detail in Geysen, European Patent Application 84/03564, published on September 13, 1984. In summary, large numbers of different small peptide test compounds are synthesized on a solid substrate, such as plastic pins or some other surface. The peptide test compounds are reacted with myrosinase fragments and washed. Bound myrosinase is then detected - such as by appropriately adapting methods well known in the art. Purified myrosinase can also be coated directly onto plates for use in the aforementioned drug screening techniques. Alternatively, non-neutralising antibodies can be used to capture the peptide and immobilise it on a solid support.

This invention also contemplates the use of competitive drug screening assays in which neutralising antibodies capable of binding myrosinase specifically compete with a test compound for binding myrosinase. In this manner, the antibodies can be used to detect the presence of any peptide which shares one or more antigenic determinants with myrosinase.

The assay method of the present invention may be a high throughput screen (HTS). In this regard, the teachings of WO 84/03564 may be adapted for the polypeptide of the present invention.

The teachings of US-A-5738985 may be adapted for the assay method of the present invention.

### MODULATION OF MYROSINASE ACTIVITY

Any measurable activity of myrosinase is a suitable candidate for the screening method of the present invention

As mentioned above, myrosinase catalyses the hydrolysis of glucosinotales. The hydrolysis products are β-D-glucose and a labile aglycone, which rapidly undergoes spontaneous rearrangement, eliminating sulphur, to yield a variety of metabolites such as isothiocyanates, thiocyanates, cyanoepithioalkanes, nitriles, amines and oxazolidine-2-thiones.

Modulation of myrosinase activity could be measure by examining changes in the rate of hydrolysis of a glucosinolate, either by monitoring the disappearance of the starting product or by monitoring appearance of one or more breakdown products.

For example, the capacity of myrosinase to hydrolyse the glycosinolate progoitrin can be measured as described in MacGibbon and Allison 1968 and 1978 (as above). Myrosinase activity can also be measured based on the determination of glucose released by the hydrolysis of 2-propenyl glucosinolate (sinigrin) as described in the Examples.

The present inventors have shown that aphid myrosinase is also capable of catalysing transglycosylation reactions, and they predict that aphid myrosinase will be capable of catalysing sulphation reactions. Modulation of either of these activities could also form the basis for the screening method.

Compounds known to be capable of modulating myrosinase activity include: polyhydroxyalkaloids such as DMDP, castanospermine and Alexine, AB1.

### MODULATION OF MYROSINASE EXPRESSION

There are numerous mechanisms known in the art by which the expression of a protein may be modulated.

The expression of a protein may be increased in a particular cell by expression of the protein itself from a heterologous promoter. For example, the cell can be transfected with a vector comprising the gene, which expresses the protein independently from expression of the endogenous gene. Alternatively, the activity or expression of one or more of the cellular components involved in controlling transcription of the gene can be modulated.

The expression of a protein can be reduced by directly interfering with transcription and/or translation of the gene, for example, by the use of antisense or ribozyme technology. In this respect, the compound may be a nucleic acid sequence capable of hybridising with the myrosinase mRNA sequence. Candidate compounds useful in the inhibition of myrosinase expression can thus be designed based on the nucleic acid sequence of myrosinase.

There are numerous methods suitable for measuring the expression of myrosinase, by measuring expression of the gene or the protein.

Myrosinase gene expression may be measured using the polymerase chain reaction (PCR), for example using RT-PCR. RT-PCR may be a useful technique where the candidate compound is designed to block the transcription of the myrosinase gene. Alternatively, the presence or amount of myrosinase mRNA can be detected using Northern blot. Northern blotting techniques are particularly suitable if the candidate compound is designed to act by causing degradation of the myrosinase mRNA. For example, if the candidate compound is an antisense sequence, which may cause the target mRNA to be degraded by enzymes such as RNAse H.

Myrosinase protein expression may be detected or measures by a number of known techniques, including Western blotting, immunoprecipitation, immunocytochemisty techniques, immunohistochemistry, *in situ* hybridisation, ELISA, radio-immunolabelling, fluorescent labelling techniques (fluorimetry, confocal microscopy) and spectrophotometry.

### AGENTS

The screening method of the present invention may be used to identify an agent capable of modulating myrosinase activity.

The agent can be, for example, an organic compound or an inorganic compound. The agent can be, for example, a nucleotide sequence that is antisense to all or part of the nucleotide sequence of the second aspect of the present invention. The agent may be selected from one of the following (non-exhaustive) list: peptide, polypeptide, oligonucleotide, polynucleotide, oligosaccharide, small organic molecule, ribozyme and antibody (or part thereof).

The methods appropriate to synthesise the identified compound will depend on its nature. For example, if the compound is a simple organic molecule, it may be synthesised using organic chemistry techniques. If the compound is a peptide, it may be synthesised using a peptide synthesiser. For longer peptides, polypeptides and proteins, it is usually easier to synthesise the compound using recombinant techniques, well known in the art. Alternatively, proteins may be isolated from source and polypeptides/peptides generated from them by protein degradation. Nucleic acids may be synthesised synthetically, or expressed from a gene. Where the successful candidate compound is a nucleic acid sequence, the compound can be synthesised by amplification from the candidate compound by known techniques (such as PCR).

The agent is capable of modulating the activity and/or expression of myrosinase. The agent may enhance the activity or expression (for example, by acting as an agonist), inhihibit the activity or expression (for example, by acting as an antagonist), or alter the nature of the activity or expression (for example, by altering the substrate specificity of the activity, or the tissue distribution of the expression).

The compounds capable of inhibiting β-glucosidases, but not plant myrosinase (such as D-glucono-γ-lactone and deoxynojirimycin) are good candidate inhibitors for aphid myrosinase. These inhibitors mimic the putative oxocarbonium ion intermediate in the reaction which has an sp² hybridised carbon at the 1-position of the sugar. Deoxynojirimycin is also protonated by the catalytic glutamic acid residue, which normally protonates the aglycone, and this increases its binding to the, enzyme. The absence of this residue from the active site of plant myrosinase could therefore explain the poor inhibition observed with this compound.

Other good candidate inhibitors for aphid myrosinase are synthetic analogues of glucosinolates. Such compounds may be useful as selective systemic insecticides.

### ANTI-CANCER TREATMENT

The present invention also provides the use of a polypeptide or a nucleotide sequence according to the invention, in the manufacture of a medicament for the treatment or prevention of cancer.

In a preferred embodiment, the method comprises the step of generating a glucosinolate and/or a glucosinolate breakdown product.

Breakdown products of myrosinase-mediated hydrolysis of glucosinolates include: β-D-glucose, aglycone, isothiocyanates, thiocyanates, cyanoepithioalkanes, nitriles, amines and oxazolidine-2-thiones.

The polypeptide and nucleotide sequences of the present invention may be used to treat and/or prevent cancer by preparation of glucosinolates and/or one or more glucosinolate breakdown products in vitro, as a step in drug production.

It has been suggested that consumption of large quantities of fruit and vegetables is associated with a reduction in the risk of developing a variety of malignancies. In a further embodiment, polypeptide and nucleotide sequences of the present invention may be used to increase the concentration of a glucosinolate and/or a glucosinolate breakdown product in a foodstuff, thereby increasing the anti-cancer effect of the foodstuff when ingested. For example, the foodstuff may be an edible plant, in particular a plant of the family *Cruciferae*, especially those of the genus *Brassica.* Edible *Brassica* plants include: cabbage, cauliflower, sprouts and broccoli.

### INCREASING DISEASE/PEST RESISTANCE

The present invention also provides a method for enhancing pest and/or disease resistance in a plant which comprises the step of expressing a polypeptide according to the first aspect of the invention in the plant.

Unlike plant myrosinase, the aphid myrosinase of the present invention does not require ascorbic acid for activation. Expression of the aphid enzyme in a plant would enable the myrosinase activity to be constitutive even in the absence of ascorbic acid.

The nucleotide of the present invention could be introduced into the plant by standard recombinant technology techniques, as described above.

Boosting the pest/disease resistance by the method of the present invention would be particularly advantageous for plants of the family *Cruciferae,* especially those of the genus *Brassica. Brassica* crops which are particularly amenable to treatment include: cabbage, oilseed rape, sprouts and broccoli.

The method of the present invention would be particularly useful for conferring resistance against pests and diseases which affect *Brassica* crops.

### INSECTICIDE

The enzyme of the present invention may be inhibited by an insecticide.

The insecticide may be useful against those insects which have an endogenous myrosinase enzyme. So far, such an enzyme is known to be present in the specialist aphids *Brevicoryne brassicae* L and *Lipaphis erysimi.* However, it may be present in other aphids or related insects.

The insecticide may be suitable for use against insects of the order Homoptera.

The order Homoptera, often regarded as a separate suborder of the order Hemiptera, includes those insects known as plant bugs. These insects have piercing and sucking mouth-parts and feed upon sap. They include the aphids [family Aphididae], white flies [Aleyrodidae], planthoppers [Delphacidae], leafhoppers [Cicadellidae], jumping plant lice [Psyllidae] woolly aphids [Pemphigidae], mealy bugs [Pseudococcidae], and scales [Coccidae, Diaspididae, Asterolecaniidae and Margarodidae].

Many spedes are serious pests of agricultural and horticultural crops and of ornamental plants, including, for example, pea aphid, black bean aphid, cotton aphid, green apple aphid, glasshouse-potato aphid, leaf-curling plum aphid, banana aphid, cabbage aphid, turnip aphid, peach-potato aphid, corn leaf aphid, wheat aphid, brassica whitefly, tobacco whitefly, glasshouse whitefly, citrus blackfly, small brown planthopper, rice brown planthopper, sugarcane planthopper, white-backed planthopper, green rice leafhopper, beet leafhopper, cotton jassid, zig-zag winged rice leafhopper, apple sucker, pear sucker, woolly apple aphid, lettuce root woolly aphid, grape phylloxera, long-tailed mealybug, pineapple mealybug, striped mealybug, pink sugarcane mealybug, cottony cushion scale, olive scale, mussel scale, San Jose scale, Califomia red scale, Florida red scale and coconut scale.

In a preferred embodiment, the insecticide is useful against aphids. In particular, the insecticide may be useful against the cabbage aphid *Brevicoryne brassicae* L and/or *Lipaphis erysimi.*

The insecticide may be capable of treating or preventing insect-associated plant damage and diseases. Crop damage as a result of feeding by insects such as those of the order Homoptera occurs in a number of ways. Extraction of sap deprives the plant of nutrients and water leading to loss of vigour and wilting. Phytotoxic substances present in the saliva of some species, and mechanical blockage of the phloem by feeding may result in distortion and necrosis of foliage [as in hopper-burn'] and in blindness or shrunken kernels in grain crops. Injury, caused by insertion of the mouthparts leaves lesions through which plant pathogens may enter. Production of copious 'honeydew' may allow sooty moulds to develop or its stickiness may interfere with the harvesting of cereals and cotton.

Some of the most serious damage caused by insects is indirect, due to their role as vectors of plant viruses. Examples of serious virus diseases spread by Homopterans include maize streak, beet curly-top, northern cereal mosaic, oat rosette, pear decline, tobacco mosaic, cauliflower mosaic, turnip mosaic, rice orange leaf, rice dwarf, rice yellow dwarf, rice transitory yellowing, rice grassy stunt, sugarcane Fiji disease, cassava mosaic, cotton leaf-curl, tobacco leaf-curl, sweet potato virus B, groundnut rosette, banana bunchy top, citrus tristeza, pineapple mealybug wilt and cocoa swollen shoot.

### TRANSGLYCOSYLATION

β-glucosidases are known to be capable of catalysing transglycosylation reactions and can give up to 96% transglycosylation under favourable conditions. It has previously been demonstrated that plant myrosinase is unable to catalyse transglycosylation reactions (M. G. Botti, M. G. Taylor and N. P. Botting, *J. Biol. Chem.,* 1995, **270,** 20530-20535).

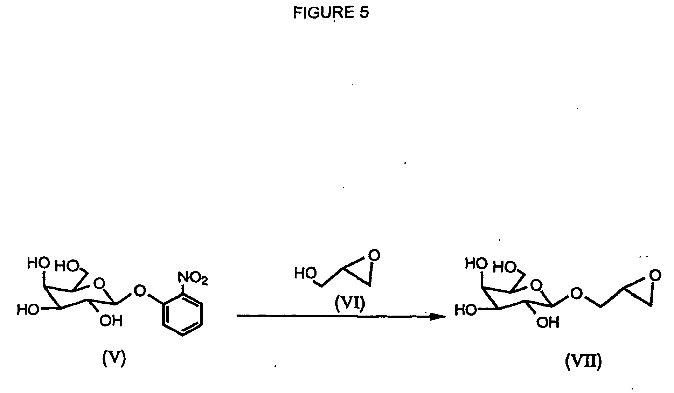

Transglycosylation can be used in the synthesis of glycosides. For example, when o-nitrophenyl-β-D-galactosidase is hydrolysed in the presence of the epoxy alcohol, the galactosyl moiety is transferred to the acceptor hydroxyl group to give the new β-galactoside (Figure 5).

The present inventors have shown that aphid myrosinase are capable of catalysing transglycosylation reactions, so the enzyme is an alternative biocatalyst for the synthesis of glycosides with charged side chains.

As used herein, the term "transglycosylation" means the transfer of residues from glycoside substratyes to acceptor molecules other than water (Dale *et al* (1986) Biochemistry 25:2522-2529; Sinnot and Vitarelle (1973) Biochem. J. 133:81-88).

### SULPHATION

The polypeptides of the present invention may be capable of catalysing a sulphation reaction. In particular, the polypeptides of the present invention may be capable of acting as carbohydrate sulfotransferases.

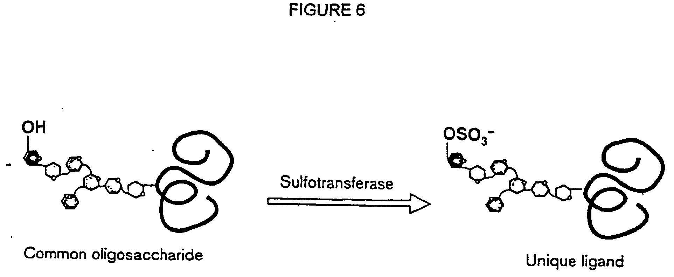

As used herein, the term "sulphation" refers to the transfer of a sulphate group (Figure 6).

There are a number of potential uses for the sulphation reaction. For example sulphation of carbohydrates may be used for generating unique ligands with specific receptor-binding activity (see Figure 6) (Hooper *et al* (1996) FASEB J. **10** 1137-1146).

The sulphated carbohydrate made by the method of the invention may be useful to treat and/or prevent an medical condition. In particular, the medical condition may be associated with HIV, HCMV infection, angiogenesis, tumor metastasis or irregularities in blood clotting.

The sulphated carbohydrate may modulate the immune response within an individual, particularly leukocyte-endothelial cell interactions, the activation state of specific members of the selectin family, L-selectin-mediated leukocyte rolling, macrophage-stimulation activity or binding to platelet-derived growth factors.

### MOLECULAR MODELLING

The enzyme of the present invention may be used to make a model for the 3D structure of aphid myrosinase.

As used herein, the term "modelling" includes the quantitative and qualitative analysis of molecular structure and/or function based on atomic structural information and interaction models. The term "modelling" includes conventional numeric-based molecular dynamic and energy minimization models, interactive computer graphic models, modified molecular mechanics models, distance geometry and other structure-based constraint models.

The crystal structure of polypeptides which, from sequence comparison, are determined to be similar to the polypeptide of the invention can be used to generate a structural model such as a three dimensional (3D) structural model (or a representation thereof) of myrosinase. Alternatively, the crystal structure may be used to generate a computer model for myrosinase.

Suitable related enzymes for which a crystal structure has been determined include plant myrosinase (from *S. alba*) and O-glucosidase from white clover (*Trifolium repens,* ICBG-pdb) both of which structures are available from the Brookhaven Data Bank.

A model for myrosinase may be generated by least squares superimposition of the co-ordinates of the known crystal structure of a related enzyme on to the myrosinase sequence.

Also, the three dimensional structure of a polypeptide may be modelled from one or more polypeptides for which the crystal structure has been solved using molecular replacement. The term "molecular replacement" refers to a method that involves generating a preliminary model of a molecule or complex whose structure co-ordinates are unknown, by orienting and positioning a molecule whose structure co-ordinates are known within the unit cell of the unknown crystal, so as best to account for the observed diffraction pattern of the unknown crystal. Phases can then be calculated from this model and combined with the observed amplitudes to give an approximate Fourier synthesis of the structure whose co-ordinates are unknown. This, in turn, can be subject to any of the several forms of refinement to provide a final, accurate structure of the unknown crystal. Lattman, E., "Use of the Rotation and Translation Functions", in Methods in Enzymology, 115, pp. 55-77 (1985); M. G. Rossmann, ed., "The Molecular Replacement Method", Int. Sci. Rev. Ser., No. 13, Gordon & Breach, New York, (1972).

Other molecular modelling techniques may also be employed in accordance with this invention. See, e.g., Cohen, N. C. et al., "Molecular Modelling Software and Methods for Medicinal Chemistry", J. Med. Chem., 33, pp. 883-894 (1990). See also, Navia, M. A. and M. A. Murcko, "The Use of Structural Information in Drug Design", Current Opinions in Structural Biology, 2, pp. 202-210 (1992).

The model may be used to screen for ligands which are capable of binding myrosinase (especially those capable of binding to or near important catalytic residues). For example a solid 3D screening system or a computational screening system could be used and test compounds may be screened through either computational or manual docking.

The present invention will now be described only by way of example, in which reference will be made to the following Figures:
Figure 1 shows the full cDNA sequence of the aphid myrosinase from *Brevicoryne brassicae* and deduced amino acid sequence. Primer positions are underlined, specific primers are doubly underlined, peptides obtained by amino acid sequencing are in bold.

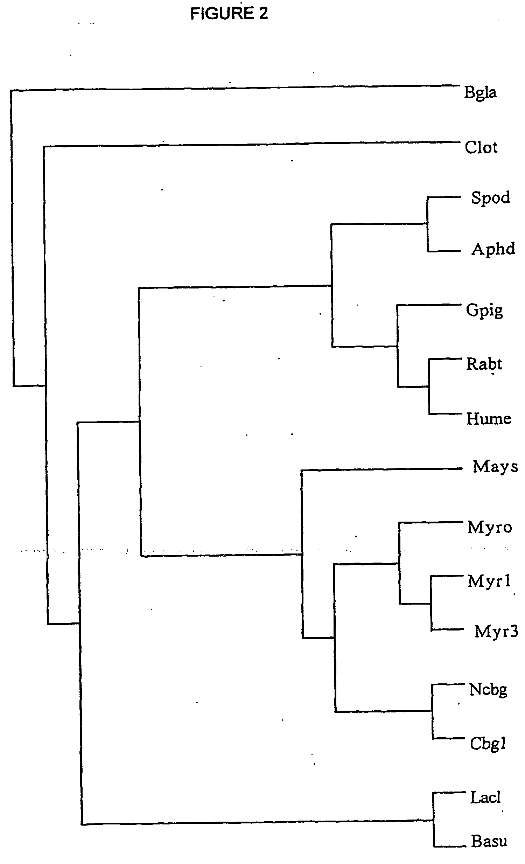

Figure 2 shows a phylogenetic tree for some members of glycosyl hydrolase family 1, constructed using the program Protpars from Phylip. LACL is a β-galactosidae from *Lactococcus lactis,* β-glucosidases are: BASU from *Bacillus subtilis,* BGLA from *Bacillus polymyxa,* MAYS from *Zea mays,* CLOT from *Clostridium thermocellum,* SPOD from *Spodoptera frugiperda,* GPIG from *Cavia porcellus.* GBG1 is cyanogenic β-glucosidase from *Trifolium repens,* NCBG is non-cyanogenic β-glucosidase from *Trifolium repens.* HUME and RABT are lactase phlorizin hydrolyses from human and rabbit respectively, Myrosinases are: MYRO from *Arabidopsis thaliana,* MYR1 and MYR3 from *Sinapis alba* APHID in myrosinase from *Brevicoryne brassicae.*
Figure 3 shows a summary of the amino acids involved in the catalysis of glucosinolates by plant (a) and aphid (b) myrosinases.
Figure 4 is a schematic representation of the metabolism of glucosinolates as catalysed by myrosinase.
Figure 5 is a schematic representation of the use of transglycosylation in the synthesis of novel galactosides.
Figure 6 is a schematic representation of the conversion of a common carbohydrate epitope (presented on a protein or lipid scaffold) into a unique ligand by installation of a sulphate ester

### EXPERIMENTAL

### METHODS

### Purification of aphid myrosinase

Freeze-dried aphids (8.7 g) were ground in extraction buffer (20 mM Tris, 0.15 M NaCl, 0.02% azide, leupeptin (10 µg/ml) and 0.1mM PMSF, pH 7.5). The extract was centrifuged at 12,000g for 30 min to remove solid matter and the supernatant fractionated with ammonium sulphate. The active fraction (40-60%) was run on a Sephacryl (S-200) gel filtration column in Tris buffer (20 mM Tris, 0.15 M NaCl, pH 7.5, 0.02% sodium azide) and active fractions pooled. The pooled fractions were mixed with 1 ml of Concanavalin A (Con A) overnight, supernatant decanted and the ConA matrix washed with buffer (2 Xs 1 ml, 20 mM Tris, 0.15 M NaCl, pH 7.5, 0.02% sodium azide) and the washings combined with the supernatant. The sample was desalted by dialysis against 10 mM imidazole (pH 6) for 2 h followed by 20 mM imidazole (pH 6) for a further 2 h. Ion exchange chromatography was carried out on a Resource Q column (Pharmacia). The column (1ml) was equilibrated with 20 mM imidazole (pH 6.5) and eluted with 20 mM imidazole (0.5 M NaCl, pH 6.5). Active fractions were pooled and desalted against starting buffer on Bio-Rad 10 DC columns. The 'main peak' sample was re-run on Resource Q and the pure protein was dialysed (2Xs) against deionised water and stored at -20 °C.

### Gel electrophoresis

Polypeptides were resolved in 12% (w/v) acrylamide vertical gel slabs according to the procedure of Laemmli (1970) with a Bio-Rad Mini Protean II electrophoretic apparatus. Polypeptides were stained with 0.25% Coomassie Blue R-250.

A narrow range IEF gel (pH 2,5 to 6.5) (Ampholine PAG precast polyacrylamide gel, Pharmacia Biotech.) was run and resolved with Coomassie Blue.

### Polyclonal antibody production

35 µg of purified aphid myrosinase was injected into a New Zealand White rabbit, followed on day 16 with 60 µg. The first bleed was taken on day 30, the terminal bleed a week later. This antibody is referred to as Wye Q. The antibodies raised to aphid myrosinase were examined for specificity by Western blotting against partially purified aphid myrosinase (from Resource Q) and against the crude protein extract from the 40 - 60% ammonium sulphate precipitate.

### Western blotting

SDS PAGE gels were run as previously described. Proteins were capillary press blotted, for 2 h, on to a nitro-cellulose membrane using 20 mM Tris, 150 mM glycine, 20% methanol (pH 8.3) as transfer buffer, at 60 °C.

### Myrosinase micro assays

An assay based on the determination of glucose released by the hydrolysis of 2-propenyl glucosinolate (sinigrin) by the aphid myrosinase was used routinely to determine enzyme activity during protein purification. GOD-Perid test reagents were purchased from Boehringer Mannheim.

Enzyme solution and sinigrin (1.08 mM) in 500 µl of sodium citrate buffer (100 mM, pH 5.5) was incubated at 30°C for 20 min. The reaction was stopped by addition of 40 µl 3M HCl (aq) and GOD-PERID reagent (2.5 ml) was added to the reaction mixture and incubated for 15 min at 37 °C. The optical density was read at 346 nm and the glucose concentration calculated from a calibration graph.

### Protein assay

Protein content was estimated using a Bradford based dye-binding kit purchased from Bio-Rad.

### Protease digests and separation of peptides

Trypsin, modified, sequencing grade (EC 3.4.21.4, Boehringer Mannheim) was used at a ratio of 1:50 (1 µg trypsin to 50 µg aphid myrosinase), in 0.2 M ammonium bicarbonate buffer, pH 7.8. Lys C (E.C. 3.4.21.50 sequencing grade Boehringer Mannheim), was used at a ratio of 1:50 (1 µg of Lys C to 50 µg of aphid myrosinase) in buffer (25 mM Tris-Cl, 1 mM EDTA, pH 8.5). The resultant peptides were separated by reverse-phase HPLC on a VYDAC, reverse-phase HPLC column (C18, 2.1mm, 15cm) using a acetonitrile/water (TFA) gradient.

### Protein Sequencing

Three peptides from the trypsin digestion and two from the Lys C digests were chosen for their apparent purity and sequenced by automated Edman degradation.

### mRNA extraction and cDNA synthesis

Total RNA was extracted from aphids using Trizol LA Reagent (Life Technologies) according to the manufacturers instructions. Dynabeads Oligo (dT)25 were purchased from Dynal and used with the buffers supplied. A cDNA Synthesis Kit was purchased from Pharmacia.

### PCR

Two degenerate primers (BmyF/R; 5'GCI TAY TAY AAY AAY YTN ATH CCN GC3', 5'CAN GGR TGN CCR AAC CAN CC3') were designed from aphid myrosinase peptide sequences and two from conserved sequences of plant myrosinases (MyrF/R: 5'TWY GTI ACI YTN TTY CAY TGG GC3', 5'GTI ARI GGN TCC ATR WAC CAN CC3'). Specific primers were designed as nucleotide sequence became available (primer positions and sequences are shown in Fig. 5).

The PCR buffer consisted of 20mM Tris-HCI, pH 8.8, 50 mM KCI, 1.5 mM Mg Cl₂ 0.25% IGPAL, primers 2 pmol/µl, 0.4 mM dNTPs. Degenerate primers were usually given a low stringency start of 4 lower temperature cycles before the annealing temperature was raised to their Tm, specific primers were run at their Tm , or just below. An average of 30 cycles were used to amplify products.

### 3' RACE

The primers were nested to reduce background amplification, as Anc3' (the 3' anchor primer) will amplify all polyA mRNA. Modifications to the standard protocol was as follows; 2 µl of the first primer set was added to the PCR mixture. The annealing temperature was 46 °C for four cycles, the annealing temperature was increased to 52 °C for a further 21 cycles. The second primer set was then added to the reaction mixture at 90 °C and the reaction run for 25 additional cycles, with annealing temperature 52 °C. A small amount of fresh Taq was added with the second primer set. The extension time was 1 min 30 s.

### 5' RACE

A 'Marathon cDNA' amplification kit (CLONETECH) was used for the following procedures. A fresh sample of mRNA was prepared according to previous protocols. cDNA was synthesised and adapters ligated according to the CLONETECH protocol. The PCR reaction mix consisted of: 35 µl MilliQ water, 5 µl 10x buffer, 2 µl dNTP mix (1 mM), 1 µl forward primer (adaptor primer), 2 µl reverse primer, 2 µl cDNA, 2 µl diluted Taq polymerase. Annealing temperature was increased to 65 °C (20 s) and the extension temperature was lowered to 70 °C for 1 min. Where nested primers were used, the secondary primers were added at 15 cycles, the total number of cycles was 30.

### DNA sequencing

Both manual and automated sequencing were used. DNA for automated sequencing was purified and desalted using QiaQuick columns. Approximately 500 ng of sample DNA was added to 4 pmol of primer and 4 µl of reaction mix. The reaction mix components were purchased from Perkin-Elmer. An ABI prism, Big Dye Terminator cycle sequencing was used with AmpliTaq DNA polymerase FS from PE Applied Biosystems. Data were analysed using ABI software from Perkin-Elmer. Manual sequencing was carried out according to standard procedures, P³³ was used to label ddNTPs.

### Antibody production

35 µg of purified aphid myrosinase was injected into a New Zealand White rabbit, followed on day 16 with 60 µg. The first bleed was taken on day 30, the terminal bleed a week later. This antibody is referred to as Wye Q. The antibodies raised to aphid myrosinase were examined for specificity by Western blotting against partially purified aphid myrosinase (from Resource Q) and against the crude protein extract from the 40 - 60% ammonium sulphate precipitate:

### Immunocytochemistry

Sections were re-hydrated by immersing the slide in PBS-GT (Phosphate buffered saline + goat serum + Tween 20) for 15 minutes. Tissue sections were blocked by covering every "well" with 5% normal goat serum in PBS-T for 30 minutes. Blocking agent was removed by shaking the slide vigorously. Primary antibody solutions were applied at a range of dilutions together with appropriate control treatments. These control treatments were : a) substitution of the primary antibody serum pre-immune serum from the same animal at the same dilution, b) substitution of the primary antibody serum with an equivalent dilution of serum which had been previously incubated with purified antigen, in order to pre-absorb antibodies to the antigen of interest, and c) complete omission of the primary antibody incubation and its replacement with buffered saline. This control treatment is important when using an indirect, two antibody staining procedure, in order to confirm the absence of nonspecific background labelling by the secondary antibody. Care was taken not to allow the control treatment droplets or the diluted antibody droplets to merge on the slide. The antibody incubation continued for 1 hour, after which the slide was rinsed by immersion in several changes of PBS-GT. A gold-conjugated secondary antibody (5 nm colloidal gold conjugated to goat anti-rabbit serum, British BioCell International) diluted 1:200 in PBS-GT was applied, 20 µl to each "well" and incubated for 1 hour. The slide was rinsed in several changes of PBS-GT, and finally in de-ionised water for 5 minutes.

Bound antibody was visualised by enhancement of the gold colloid with nucleated silver (IntenSE M silver enhancement kit, Amersham Life Sciences) The technique involves incubation of the slide in freshly prepared reagent. Enhancement was monitored using a standard binocular microscope and was stopped by rinsing the slide in several changes of de-ionised water, for at least 5 minutes. Close monitoring of the enhancement was necessary to allow sufficient intensification of bound antibody complexes, while avoiding self-nucleation of the enhancement reagent which occurs after extended periods of incubation. After enhancement, tissue was counterstained by brief immersion of the slide in 0.001% toluidine blue in 0.001% borax, warm air dried, and mounted using a standard histological mountant. All incubation steps were carried out in a humid chamber at 37° C.

### Electron microscopy

Sections were rehydrated by immersion of grids in 20 µl droplets PBS-GT for 15 minutes, transferred to blocking solution (5% normal goat serum in PBS-GT) and incubated for 30 minutes. Grids were removed from blocking solution and immersed into diluted primary antibody and incubated for at least 1 hour. A range of primary antibody control treatments were also included. Grids were rinsed by passage through a series of 50 µL droplets of PBS-GT (excess solution was removed from each grid, between steps, by touching the edge of the grid to a folded filter paper "blotter") and incubated in secondary antibody (20 or 30 nm colloidal gold conjugated to goat anti-rabbit serum, British BioCell International) diluted 1: 200 in PBS-GT for 1 hour. All incubation steps were performed in a humid chamber (Parafilm sheet on wet filter paper enclosed in a polystyrene box) at 37°C. Grids were gently rinsed in deionised water, stained in saturated aqueous uranyl acetate for 35 minutes, and Reynolds lead acetate (Reynolds 1963) for 3 minutes (in a CO₂ - free environment). After repeated rinsing in de-ionised water, specimen grids were air dried and stored in a dry, dust-free environment until viewing using an Hitachi H-7000 transmission electron microscope with acceleration voltage set at 75 kV.

### Molecular modelling

Five templates (cyanogenic β-glucosidase, PDB-1CBG; plant myrosinase, PDB-1MYR; plant myrosinase complexed with 2-deoxy-2-fluoro-glucosyl, PDB-2MYR; β-glucosidase A, PDB-1BGA; β-glucosidase A complexed with gluconate, PDB-1 BGG) were used to generate the 3D model for aphid myrosinase while all postulated functions of amino acid residues come from Burmeister et al. (1997) *Structure.* 5: (5) 663-675.

### Kinetic studies

An assay based on the determination of glucose released by the hydrolysis of 2-propenyl glucosinolate (sinigrin) by the aphid myrosinase was used routinely to determine enzyme activity during protein purification. GOD-Perid test reagents were purchased from Boehringer Mannheim.

Enzyme solution and sinigrin (1.08 mM) in 500 µl of sodium citrate buffer (100 mM, pH 5.5) was incubated at 30°C for 20 min. The reaction was stopped by addition of 40 µl 3M HCl (aq) and GOD-PERID reagent (2.5 ml) added to the reaction mixture and incubated for 15 min at 37 °C. The optical density was read at 346 nm and the glucose concentration calculated from a calibration graph.

### EXAMPLE 1 - Purification

The myrosinase from freeze-dried aphids was purified in five steps (Table 1). Myrosinase was precipitated at 40 - 60% saturation with ammonium sulphate with no appreciable activity present in any other fractions. The gel filtration step (Table 1) yielded a four-fold purification while affinity chromatography on Concanavilin A removed glycosylated proteins resulting in further purification. Aphid myrosinase did not bind to the lectin concanavalin A indicating that either the protein is not glycosylated or its glycosyl component is not specific for this type of lectin. Ion exchange chromatography, on a Resource Q column gave a major and minor peak of aphid myrosinase activity which were resolved by fractionation and subsequent rechromatography resulting in a single homogenous peak. Characterisation of the minor aphid myrosinase peak was not attempted as there was insufficient material. Although the specific activity of the sample increased total activity declined during this step. Overall, the total purification achieved was forty-fold, while the total yield of protein was 0.13% of the crude extract. The purity of the protein extract was assessed by SDS-PAGE and comparison with BSA and isoelectric focusing (see Example 2).

### EXAMPLE 2 - Initial Characterisation

The native molecular mass of aphid myrosinase, estimated from gel filtration, was 97 kDa. The molecular mass of the denatured and reduced protein was 53 kDa, estimated from SDS PAGE. The molecular mass of the subunit was confirmed by MALDI-TOF mass spectrometry, giving a value of 54,415 Da. Thus aphid myrosinase appears to be a dimeric protein, with identical subunits.

Isoelectric focusing of the purified aphid myrosinase gave two bands. The isoelectric point (pl) of these bands were 4.90 and 4.95 the latter being considerably denser then the former. The less dense band observed with a pl of 4.90 is possibly the minor peak observed in the first Resource Q ion exchange chromatography step and possibly represents an isoform of aphid myrosinase.

The pH optima of the enzyme was found to be 5.5 compared to a previously reported pH optima of 5 (MacGibbon_DB and Allison_RM, NZ J Sci, 1968. **11**: p. 440) for a crude protein extract of aphid myrosinase.

Western blots showed that the antibody raised to aphid myrosinase (Wye Q) was highly specific to a single band in crude extracts of *B. brassicae* from SDS PAGE gels. Wye Q did not cross react with proteins (also using Western blotting techniques) from *S*. *alba* and did not show a reaction to proteins from other *Brassica* pests tested (data not shown). Anti-plant myrosinase antibodies did not cross react with *B*: *brassicae* proteins. However, there was a cross reaction with the anti-plant myrosinase antibodies and herbivorous insects, which was probably due to ingested plant material. The results of the Western blots are summarised in Table 2.

### EXAMPLE 3 - Amino Acid Sequence Analysis

The intact protein was N -terminally blocked and sequence data was obtained from peptide fragments. Typsin digestion gave three peptides. Peptide A (¹LVTFGSDPNnNFNPD¹⁵) failed to match any known proteins while peptide B (¹GIAYVNNLlpELlK¹⁴) matched β-glucosidases and peptide C (¹GWFGHPVYK⁹) matched at low astringency, an apoprotein from photosystem II and various lactases which show some similarity with myrosinase (Manntei_N*, et al.,* EMBO, 1988. 7(9): p. 2705-2713). Lys C digestion gave two peptides. Peptides D (¹TTGHYLAGHT¹⁰) and E (¹ISYLK⁵) did not match any known protein with any degree of probability.

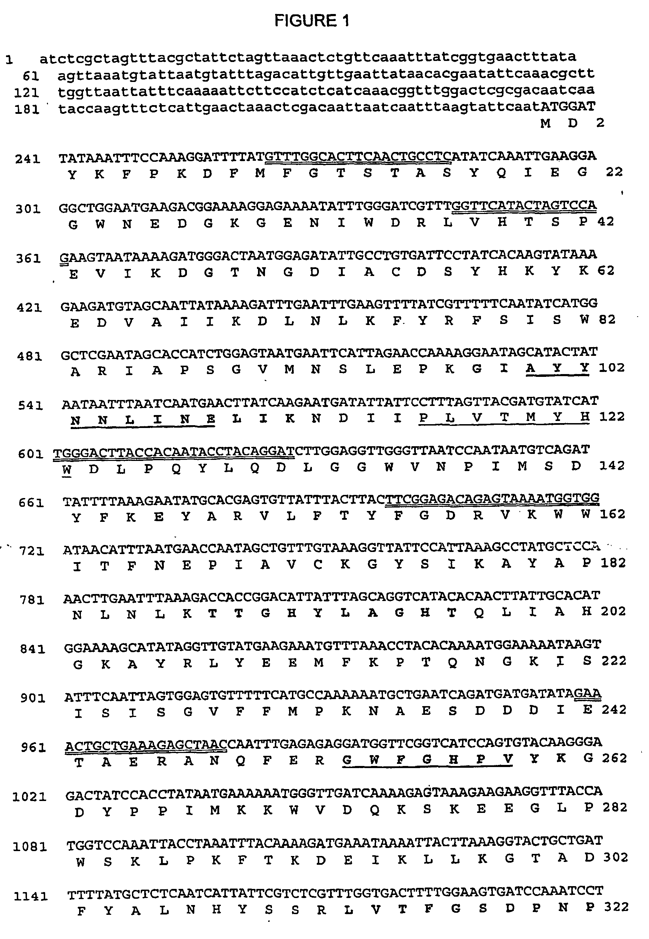

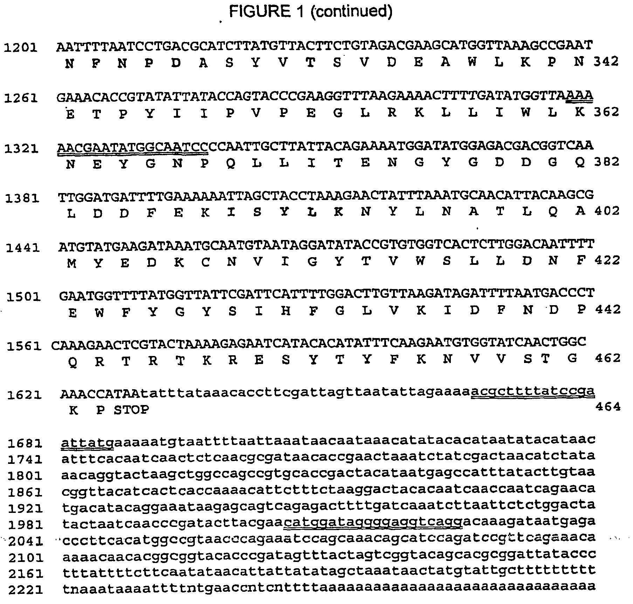

The full cDNA sequence of aphid myrosinase is shown in Figure 1. All sequenced peptides could be deduced from the cDNA sequence. A search of the 'Blocks' database (Henikoff S. and Henikoff JG., Genomics, 1994. **19:** p. 97-107) showed that aphid myrosinase has six motifs belonging to glycosyl hydrolase family 1 (GHF1) (Henrissat B, Biochem J., 1991. **280**: p. 309-316) (Table 3).

A search of the ProSite motif database showed one glycosylation site, two myristolylation sites and the N-terminal signature of GHF1.

A classification of glycosyl hydrolases based on amino acid sequence similarities was established (Henrissat B as above) which should reflect the structural features of these enzymes better then their substrate specificity alone. Further more evolutionary relationships may be revealed by this system as the three dimensional folding of enzymes is more highly conserved than their sequences (Chothia C. Nature, 1992. 357: p. 543-544.) BLAST sequence similarity search results showed multiple matches with β-glucosidases from various sources and with plant myrosinases. Aphid myrosinase shows significant sequence similarity to plant myrosinases (35%) and other members of the glycosyl hydrolase family 1 (GH1). Greatest similarity was with the β-glucosidase from *Spodoptera frugiperda* followed by the lactase phlorizin hydrolases, all belonging to GHF1 (Table 4).

### EXAMPLE 4 - Production of an antibody to aphid myrosinase and localisation studies

An polyclonal antibody was raised to the aphid myrosinase using standard immunisation techniques.

The localisation of the myrosinase enzyme in the aphid was determined by immunocytochemistry and electron microscopy. The enzyme was found to be located in the muscle of the head and the thorax and is present as regular crystal-like structures.

### EXAMPLE 5 - Molecular modelling of aphid myrosinase

The 3D structures of myrosinase (*S*.*alba* 1 yr. pdb) and the O-glucosidase from white clover *(Trifolium repens,* ICBG-pdb) are available from the Brookhaven Data Bank and were used as templates in homology modelling of aphid myrosinase. All members of GH1 share the same fold, namely a TIM barrel in which the catalytic residues are located (Davies and Henrissat (1995) Structure **3**: 853-859) Hydrolysis of glucosides proceeds by general acid base catalysis using two glutamate residues (as proton donor and nucleophile). Myrosinases differ from other members of this family in that one of the glutamate residues has been replaced by a glutamine as the second glutamate is not required for catalysis due to the superior leaving group properties of the glucosinolate side-chain.

Molecular modelling of aphid myrosinase using the sequence information along with examination of the sequence similarity has allowed the present inventors to identify the putative active site and catalytic residues. Sequence comparisons have shown that the enzyme resembles both plant myrosinases and some O-glucosidases (see below). The active site appears to be a hybrid of the two sorts of enzyme, with catalytic machinery more like an O-glucosidase and substrate binding motifs like the plant myrosinases, possibly implying that it is an O-glucosidase which has evolved myrosinase-like activity.

Intriguingly, aphid myrosinase appears to possess both glutamate residues in common with O-glucosidases, from which it is suggested that this enzyme has evolved. The two glutamates are Glu314, the catalytic nucleophile, and Glu 167 which appears to be responsible for protonation of the aglycone. Close examination of the structure did not reveal two suitably positioned arginine residues for binding to the sulfate as is observed in the plant enzyme. However other candidates have been identified that could play a role. In the aphid enzyme Lys173 and Arg312 seem to be in a suitable position for binding the sulfate and both would be positively charged at physiological pH. Interestingly both the plant and aphid myrosinases seem to have a number of basic residues clustered around the periphery of the active site, but these are not present in the clover β-glucosidase.

Aphid myrosinase is a globular protein, of about 50 A in diameter with a cleft into the core of the structure where the putative active site residues are located. The superimposition of the α-carbon skeleton of aphid myrosinase onto plant myrosinase and cyanogenic β-glucosidase showed that their main structure was very similar. A loop consisting of residues 270 to 280 was found in aphid myrosinase but is absent in both cyanogenic β-glucosidase and plant myrosinase. This loop occurs on the outer part of the aphid myrosinase, appearing to fold into two anti-parallel β-sheets. However, as this structure is not found in the templates and its location indicates little steric hinderance to various conformations, it is the least reliable part of the model.

The validity of the model was assessed using the SwisspbdViewer molecular modelling program. A root mean square deviation of 3.26 Å was obtained for the superimposition of the α carbon skeleton of aphid myrosinase onto plant myrosinase. Using Predictprotein (from the Expasy site) a z-score of 1.6 was obtained, and since a value of 4.5 gives correct predictions in 88% of test cases (Rost *et al.,* 1995 J. Mol. Biol. 270:471-480) the model was considered to be accurate. For the purposes of comparison the postulated role of the amino acid residues in plant myrosinase, cyanogenic β-glucosidase and aphid myrosinase are shown in Table 5.

The residues acting as proton donor and nucleophile, in the hydrolysis of glucosinolates by aphid myrosinase, are identified as Glu 167 and Glu 374 respectively. The equivalent residues in plant myrosinase are Gin 187 and Glu 409 and superimpose well with Glu 167 and Glu 374 of the aphid myrosinase. The equivalent for the cyanogenic β-glucosidase is Glu 183 and Glu 397.

The recognition of the glucose ring is mediated by six hydrogen bonds in plant myrosinase. The residues involved are: Glu 464, Gln 39, His 141, Asn 186. Recognition of the glucose ring occurs in a hydrophobic environment formed by residues Tyr 330, Trp 457, Phe 465 and Phe 473 in plant myrosinase. The equivalent residues in aphid myrosinase and cyanogenic β-glucosidase are shown in Table 5. These residues are identical in all three enzymes, except for the replacement of Phe 465 of myrosinase with Trp in aphid myrosinase and cyanogenic β-glucosidase. This substitution would minimally effect the hydrophobicity of the surrounding area. These residues are highly conserved in all members of GHF1 and are considered to be highly specific to the glycone moiety of their substrates (Dey, 1987 Adv Erizolo 56:141-249).

In the native structure of plant myrosinase, Glu 409 forms a salt bridge with Arg 95. This salt bridge is disrupted on formation of the glycosyl-enzyme and the side chain of Glu 409 changes its conformation and the charge of Arg 95 becomes buried (Burmeister *et al.,* 1997Structure 5:663-675). Arg 95 (plant myrosinase) corresponds to Arg 77 in aphid myrosinase and the two superimpose extremely well.

These arginine residues are found in a highly conserved motif in GHF1 and it is possible that the disruption of this salt bridge is common to all members of this family.

Two features specific to plant myrosinase were identified by Burmeister *et al*. (1997) (as above) by comparison with cyanogenic β-glucosidase. The first is a hydrophobic pocket for the aglycone moiety of glucosinolates and the second are residues which recognise the sulphate group of glucosinolates. The hydrophobic pocket is formed by residues: Phe 331, Phe 371, Phe 473, lie 257 and Tyr 330 in myrosinase. Aphid myrosinase possess residues: Ser 310, Tyr 346, Phe 432, Ser 226 and Tyr 309 (respectively) in these positions. Equivalent residues in cyanogenic β-glucosidase are shown in Table 5. Residues equivalent to Phe 473 and Tyr 330 are common in members of GHF1, as these residues form part of the hydrophobic pocket important in recognition of glucose. Serine residues are hydrophilic and are unlikely to contribute to a hydrophobic environment. Phe 371 (plant myrosinase) and Tyr 346 (aphid) do not superimpose well and occur in highly variable parts of the enzymes, which are hard to align correctly as they are proline rich. BLAST identifies this region as one of low complexity. Furthermore aphid myrosinase has several deletions in this area, so the model may not be entirely correct here. However, hydrophobic cluster analysis (see below) reveals a hydrophobic residue near to the nucleophile in plant myrosinase (IIe 412) and aphid myrosinase (Tyr 377) but not in cyanogenic β-glucosidase (Arg 380). These residues are near to the area occupied by the aglycone and may contribute towards the formation of a hydrophobic pocket in both plant and aphid myrosinases.

Recognition of the sulphate group of glucosinolates is probably mediated by residues Arg 194 and Arg 259 within a positive pocket in plant myrosinase. In aphid myrosinase Lys 173 and Val 228 are similarly positioned and its possible that Lys 173, but not Val 228 may play a similar role. In addition the basic residue Arg 312 is located in the active site and may contribute to recognition of the sulphate group. In cyanogenic β-glucosidase Asn 190 and His 256 are found in equivalent positions. Although Lys 173 appears to point away from the active site in aphid myrosinase, the side chain of this residue can be rotated. In alignments of the enzymes it is noticeable that aphid myrosinase has a deletion just before Lys 173 and the occurrence of a basic residue in this position is found only in myrosinases.

In plant myrosinase, Ser 190 defines the position of Gin 187 and also hydrogen bonds to the sulphate group. Burmeister *et al.* (1997) (as above) state that the hydrogen bond between Ser 190 (Oγ) and Gin 187 (*N*ε2) is a feature found only in myrosinases. Aphid myrosinase possesses an Ala (170) residue in place of Ser 190 (and Glu 176 in place of Gln 187) and would be unable to form this hydrogen bond. Perhaps, as the proton donor (Glu 167) is found in aphid myrosinase, this hydrogen bond is not necessary. Trp 142 in plant myrosinase is in van der Waals contact with the sulphur atom of the thioglucosidic bond. Trp 123 occurs in aphid myrosinase but this Trp is common to members of GHF1 and need not be a specific feature. Gln 187, of myrosinase, may play a specific role in the hydrolysis of glucosinolates, this residue can hydrogen bond to the sulphate of the aglycone. A glutamate reside in this position may cause unfavourable electrostatic interactions (Burmeister *et al*., 1997, as above).

### EXAMPLE 6 - Hydrophobic Cluster Analysis

The results of hydrophobic cluster analysis support the similarities of three dimensional structure observed in myrosinase, cyanogenic β-glucosidase and aphid myrosinase. The motifs described by Henrissat *et al.* (1995) (Proc. Acad. Natl. Sci. 92:7090-7094) are present in all three enzymes, as would be expected. The first motif consists of a large horizontal cluster (which corresponds to a helix) and a short vertical cluster (strand) followed by a Asn-Glu dipeptide, the Glu is the acid catalyst The second motif is a short vertical cluster which preceeds the nucleophile (Glu). These motifs were found in more than 150 glycosyl hydrolase sequences and common ancestory was proposed for this group (Henrissat *et al*., 1995, as above).

HCA revealed a large hydrophobic cluster prior to the proton donor in aphid myrosinase which is more similar to that found in plant myrosinase than cyanogenic β-glucosidase. Near to the nucleophile hydrophobic residues are found in aphid myrosinase (Tyr 377) and myrosinase (IIe 412) but not in cyanogenic β-glucosidase (Arg 380).

### EXAMPLE 7 - Phylogenetic analysis

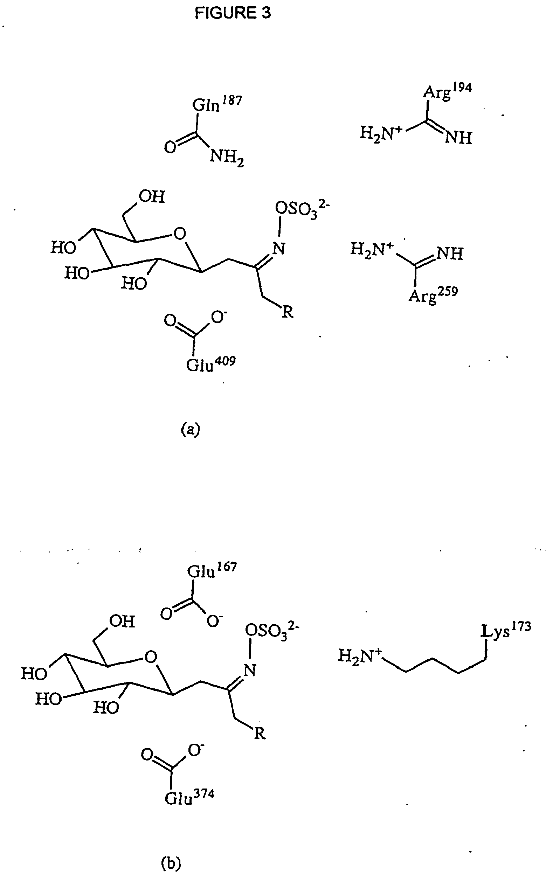

The alignment of aphid myrosinase was based against fourteen members of GHF1. This alignment was used in phylogenetic analyses. An unrooted phylogenetic tree was constructed based on maximum parsimony techniques, using the Phylip program Protpars (Fig 2). Aphid myrosinase clearly groups with the animal β-glucosidases and lactase phlorizin hydrolases (LPHs), being most similar to the β-glucosidase from the fall armyworm, *Spodoptera frugiperda* (SPOD). Myrosinases cluster together with β-glucosidases from white clover, *Trifolium repens,* and maize, *Zea mays.* Thus, it would appear that the ability to hydrolyse glucosinolates has arisen separately in plant and animal β-glucosidases. Aphid myrosinase appears to be more similar to animal β-*O*-glucosidases than to plant myrosinases, as assessed by sequence similarity and phylogenetic techniques. These results strongly suggest that myrosinase activity has arisen twice from *O*-glucosidases in plants and animals. The active site of aphid myrosinase is similar to β-*O*-glucosidases as both proton donor and nucleophile are present and the possible interactions of glucosinolate and proton donor and nucleophile are shown in Fig. 3.

### EXAMPLE 8 - Kinetic studies

Polyhydroxyalkaloids which inhibit glycosidases from a wide range of organisms and are believed to play a role in plant defence against herbivory (Fellows *et al.* 1989 Recent advances in phytochemistry 23). Scofield *et al.* (1990, Phytochemistry 29:107-9) compared the effect of seven of these alkaloids on myrosinases from the mustard plant *Brassica nigra,* and the cabbage aphid *B. brassicae,* to extend previous work on *O*-glucosidases to *S*-glucosidases. Using the glucosinolates 2-propenyl and 2-hydroxy-3-butenyl glucosinolate it was shown that DMDP, an analogue of β-D-fructofuranose, [(2*R*, 5*R*)-dihydroxymethyl-(3*R*,4*R*)-dihydroxypyrrolidine] and castanospermine effectively inhibited the myrosinases from both plant and aphid. Alexine, AB1 [1,4-dideoxy-1,4-imino-D-arabinitol] and DNJ [1-deoxynojirimycin] inhibited the aphid enzyme but not significantly plant myrosinase. The present inventors have performed a number of kinetic studies on the myrosinase from the cabbage aphid *(Brevicoryne brassicae)* which they have characterised. The apparent Km of the aphid myrosinase was 0.613 and 0.915 mM respectively for 2-propenylglucosinolate and benzyl glucosinolate indicating that the enzyme has a greater affinity for 2-propenylglucosinolate, a common glucosinolate in cabbage and mustard plants. Like the cabbage aphid, the turnip aphid (*Lipaphis erysimi* ) myrosinase was not activated by ascorbate in the concentration range 0.1-20 mM. The K_{M} for sinigrin is 0.42 mM for the white mustard *(Sinapis alba)* myrosinase.

The aphid enzyme was competitively inhibited by 2-deoxy-2-fluoroglucotropaeolin (MacGibbon and Allison 1978, as above) with an extremely low Ki, suggesting more than simple competition and that a stable glycosyl-enzyme is produced in the same way as for the plant enzyme.

### EXAMPLE 9 - Cloning, Overexpression and Purification of Aphid Myrosinase

The full cDNA sequence of aphid myrosinase has been obtained by 5'-RACE and 3'-RACE using a CLONTECH Smart cDNA Amplification kit. Primers were based on the amino acid sequence of purified peptides from trypsin digests of purified aphid myrosinase. A full length cDNA can be obtained by digestion of the cloned 5' and 3' RACE fragments at appropriate overlapping restriction sites, ligation and cloning into pBluescript. The splice junctions are checked by sequencing.

In more detail, aphid myrosinase cDNA is amplified using N*d*el-tailed forward printer (5' ATT CCA TAT GGA TTA TAA ATT TCC '3 (AphMyr1F, position 228) and *Xh*ol-tailed reverse primer (5' TAT AAC TCG AGT GGT TTG CCA GTT GAT ACC '3 (AphMyr1R, position 1608)) from aphid mRNA (isolated using oligodT coated DYNAbeads) and inserted at the *C*-terminal of intein tag in pTYB12 vector (NEB) between Ndel and Xhol RE sites. pTYP12 is provided by the New England Biolab, IMPACT protein overexpression system. The aphid myr cDNA insert used is 1.38kb (starting at ATG codon) and pTYB12 vectorsize 7.42 kb, respectively.

After cloning and transformation into *E.coli* aphid myrosinase protein is produced in ERZ566 strain. Myrosinase enzyme activity is monitored as glucose released from hydrolysis of the glucosinolate sinigrin (GOD-perid assay) and is not detected in bacteria carrying control plasmid. The activity per mg protein was, however, low.

Mysrosinase protein is produced by expression of the cDNA in a yeast *(Pichia)* system or a baculovirus-insect cell system, both allowing the production of large amounts of post translationally modified protein.

The *Pichia* (a methylotrophic yeast), system allows the simplicity of *E*. *coli* expression systems with the advantages of a eukaryotic high level expression system. The EasySelect system from Invitrogen is used. Briefly, PCR amplified mysrosinase cDNA is cloned into pPICZ or an equivalent vector which contains the inducible *AOX1* promoter for high level expression in *Pichia pastoris* and transformed into *P. pastori.* Cultures are assayed for myrosinase activity by immunoblot analysis using an anti-aphid myrosinase antibody to confirm the identity of the protein, and the protein is purified from methanol induced cultures of *P. pastori* by column chromotography using the 6xHis-tag and ProBond resin or with anti-aphid myrosinase antibodies.

In more detail, aphid myrosinase cDNA (1.38 kb) was amplified using forward primer (5' GTA GCT CGA GTG GAT TAT AAA TTT CCA 3' (Pp1FXhol, position 226) and reverse primer (5' TAT GGA TCC CTT AAT GGT GAT GAT GGT GAT GTG GTT TGC CAG TTG ATA CC 3' (AphPphis - containing 6 his resdues, position 1608)) from aphid mRNA and inserted between the *Xho*I and *bam*H1 site of pHIL-S1 (Invitrogen) *P.pastoris* expression vector (8.3 kb). First codon Met was substituted with Val to keep *Xho*I site and PHO1 signal peptide encoding sequence of pHIL-S1 intact.

The alternative, baculovirus, system has the advantage is that it is an insect system. The MaxBac (Invitrogen) System is used which is very efficient, and enables easy recombinant selection and protein purification. Briefly, PCR amplified mysrosinase cDNA is cloned into the transfer vector (pBlueBac4.5/V5-His-TOPO) and transformed into TOP10 *E. Coli.* Recombinant DNA will be used with linear viral DNA (Bac-N-Blue) to co-transfect Sf9 cells. Recombinant (blue) plaques are selected and a pure clone of the recombinant virus is obtained by further rounds of plating. Individual plaques are assayed for myrosinase activity as above. The virus titre is increased by transfection of shaken cultures of Sf9 cells and the recombinant protein is purified using the 6xHis-tag and ProBond resin or by immunoaffinity column chromotography with anti-aphid myrosinase antibodies.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, biology or related fields are intended to be within the scope of the following claims.

**Table 1 Purification of aphid myrosinase from Brevicoryne brassicae.**

| **Purification step** | **Protein** (mg) | **Total activity** (µmol/min) | **Specific activity** (µmol/mg/min) | **Yield (%)** | **Purification** |
|---|---|---|---|---|---|
| Crude extract | 498.00 | 238.0 | 0.478 | 100.00 | 1.00 |
| (NH₄)₂SO₄ cut | 132.00 | 97.0 | 0.737 | 26.00 | 1.54 |
| S-200 | 20.00 | 36.0 | 1.850 | 4.00 | 3.87 |
| Con A | 10.00 | 44.0 | 4.300 | 2.00 | 9.00 |
| Res Q (I) | 1.00 | 13.0 | 13.000 | 0.20 | 27.20 |
| Pure aphid myrosinase | 0.66 | 132 | 20.000 | 0.13 | 41.84 |

**Table 2 Summarising the results of Western blots with anti-plant-myrosinase antibodies and the anti-aphid aphid myrosinase antibody. Wye Q was raised against aphid myrosinase, Wye E, D and DCJ are all raised against plant myrosinases. + indicates a positive reaction, - indicates a negative reaction. * indicates that this combination was not tested.**

| **Organism** | **Antibody used** | | | |
|---|---|---|---|---|
| **- pests** | **Wye Q** | **Wye E** | **Wye D** | **DCJ** |
| *Brevicoryne brassicae* | + | - | - | - |
| *Myzus persicae* | *-* | ******* | ******* | *** |
| *Phedon cochleariae* | - | + | - | + |
| *Peris rapae* | - | + | + | + |
| *Peris brassicae* | - | + | + | - |
| **- Plant** | | | | |
| *Sinapis alba* | *-* | *** | *** | * |

**Table 3 Results from the Blocks database showing matches to members of glycosyl hydrolase family one. Residues with matching identity are shown in bold. The enzymes to which aphid myrosinase is matched are shown for example only, standard Swissprot codes are used.**

| **Block** | **Matched to** | **Match** |
|---|---|---|
| **A** 'EGQ'motif | BGLS CALSA aphid | FPKGFLWGAATASYQIEGAWNEDGKGESIW FPKDFMFGTSTASYQIEGGWNEDGKGENIW |
| **B** 'RRP' motif | BGLS TRIRP aphid | DQYHRYKEDVGIMKDQNMDSYRFSISWPRILPKG DSYHKYKEDVAIIKDLNLKFYRYSISWARIAPSG |
| **C** 'TLH' motif | BGLS TRIRP aphid | NHEGIKYYNNLINELLANGIQPFVTLFHWDLPQVL EPKGIAYYNNLINELIKNDIIPLVTMYHWDLPQYL |
| **D** 'END' motif | BGLB MICBI aphid | LIITENGAAFDD LLITENGYGDDG |
| **E** 'GWD'motif | BGLA CLOTM aphid | DGVNLKAYYLWSLLDNFEWAYGYNKRFG DKCNVIGYTVWSLLDNFEWFYGYSIHFG |

**Table 4 Percentage similarity of aphid myrosinase compared to some members of glucosyl hydrolase family 1.**

| **Name** | **Identity (%)** | **Consertive substitution (%)** | **Gaps (%)** |
|---|---|---|---|
| β-Glucosidase precursor *Spodoptera frugiperda* | 46 | 65 | 2 |
| Lactasse phlorizin hydrolase *Oryctolagus cuniculus* (405/1926) | 43 | 62 | 2 |
| | 43 | 61 | 2 |
| | 36 | 57 | 2 |
| | 28 | 46 | 11 |
| Lactase phlorizin hydrolase *Homo sapiens* (403/1927) | 42 | 59 | 1 |
| | 41 | 60 | 2 |
| | 37 | 56 | 4 |
| | 28 | 43 | 15 |
| Gentiobiase *Clostridium thermocellum* | 40 | 58 | 6 |
| Cytosolic β-glucosidase *Cavia porcellus* | 39 | 59 | 1 |
| Cyanogenic β-glucosidase *Trifolium repens* | 43 | 58 | 8 |
| Non-cyanogenic β-glucosidase *Trifolium repens* | 36 | 52 | 9 |
| Myrosinase precursor *Brassica napus* | 34 | 52 | 12 |
| Myrosinase precursor *Sinapsis alba* | 35 | 53 | 10 |
| Klotho protein *Mus musculus* | 36 | 53 | 11 |
| | 32 | 49 | 10 |
| β-glucosidase, chloroplast precursor *Zea mays* | 38 | 54 | 14 |

**Table 5 Summary of positions and postulated functions of residues mentioned in text, the postulated function may not apply to bracketed residues**

| **Amino acid residue positions** | | | **Postuated role*** |
|---|---|---|---|
| **Plant Myrosinase (MYR)** | **Cyanogenic β-glucosidase (CBG)** | **Aphid Myrosinase (AMYR)** | |
| Gln 39 | Gln 32 | Gln 19 | H bonds to sugar ring |
| His 56 Asp 70 | (His 53) (Asp 66) | His 39 Asp 52 | Zinc²⁺ binding, dimer formation (CBG does not dimerise) |
| Arg 95 | Arg 91 | Arg 77 | Hydrophobic pocket; forms salt bridge with nucleophile (E 409, MYR) |
| His 141 | His 137 | His 122 | Hydrophobic pocket; H-bonds to inhibitor (recognition O of sugar) and Asn 186 (MYR) |
| Trp 142 | Trp 138 | Trp 123 | Sulphur recognition; in van der Waals contact with S of thioglucosidic bond (residue common in GHF1) |
| Asn 186 | Asn 182 | Asn 166 | Hydrophobic pocket; H-bonds to Arg 95 (MYR) and to sugar |
| (Gin 187) | Glu 183 | Glu 167 | Acid catalyst in β-glucosidases |
| Ser 190 | (Gly186) | (Ala 170) | Sulphur recognition; defines position of Glu 409 (MYR) and probably H-bonds to S of glucosinolate sidechain, possibly involved in hydrolysis of glycosylenzyme |
| Arg 194 | (Asn 190) | Lys 173 | Sulphur recognition |
| Ile 257 | (Val254) | Ser 226 | Hydrophobic pocket, H bonds to Glu 167 (AMYR) |
| Arg 259 | (His 256) | (Val228) | Sulphur recognition |
| Asn 328 Gln 333 | Asn 324 Tyr 329 | Asn 307 Arg 312 | Hydrophobic pocket; H-bonds to Gln 187 (MYR), possibly involved in hydrolysis of glycosyl-enzyme |
| Tyr 330 | Tyr 326 | Tyr 309 | OH to O of sugar ring |
| Phe 331 | (Ser 327) | (Ser 310) | Hydrophobic pocket |
| Phe 371 | (Ala 365) | (Glu 346) | Hydrophobic pocket? - far from active site, not present in CBG, AMYR |
| Glu 409 | Glu 397 | Glu 374 | Active site; nucleophile, highly conserved in glycosyl hydrolase family 1 |
| Ile 412 | (Arg 380) | Tyr 377 | Hydrophobic pocket |
| Trp 457 | Tcp 446 | Trp 416 | Hydrophobic pocket; under glucose ring, Nε H-bonds to inhibitor (MYR) |
| Glu 464 Phe 465 | Glu 453 Trp 453 | Glu 423 Trp 424 | Both O H-bond to inhibitor, in hydrophobic pocket |
| Phe 473 | Phe 462 | Phe 432 | Hydrophobic pocket van der Waals with sugar ring |

## Claims

1. A myrosinase enzyme from a non-plant source comprising the amino acid sequence shown in SEQ ID No. 1, or a homologue thereof having at least 75% sequence identity, which displays myrosinase activity, and which does not require ascorbic acid for activation.

2. A nucleotide sequence which encodes an enzyme according to claim 1.

3. A nucleotide sequence according to claim 2, comprising the nucleic acid sequence shown in SED ID No. 2 or:
(i) a homologue thereof having at least 75% sequence identity;
(ii) a fragment thereof which has one or more bases missing, but which retains the capacity to encode an enzyme according to claim 1; or
(iii) a derivative thereof which has a chemical modification of the side chains and/or the backbone of the nucleotide sequence but which retains the capacity to encode an enzyme according to claim 1.

4. A vector comprising the nucleotide sequence according to claim 2 or 3.

5. A host cell into which has been incorporated the nucleotide sequence according to claim 2 or 3.

6. An organism into which has been incorporated the nucleotide sequence according to claim 2 or 3, and which is a fungus, yeast, plant or prokaryotic organism.

7. An organism according to claim 6, wherein the organism is a plant.

8. An antibody which recognises the enzyme of claim 1.

9. A method for screening for an agent capable of modulating myrosinase activity and/or expression, which comprises the following steps:
(i) contacting an agent with a enzyme according to claim 1 or a nucleic acid according to claim 2 or 3;
(ii) measuring the activity and/or expression of myrosinase
wherein a difference between a) myrosinase activity/expression in the absence of agent, and b) myrosinase activity/expression in the presence of agent is indicative that the agent is capable of modulating myrosinase activity and/or expression.

10. A method according to claim 9, wherein the agent is capable of inhibiting or blocking the activity and/or expression of the myrosinase.

11. A method according to claim 10, wherein the agent is capable of inhibiting or blocking the activity and/or expression of cabbage aphid myrosinase.

12. The use of an enzyme according to claim 1 or a nucleotide sequence according to claim 2 or 3, in the manufacture of a medicament for the treatment or prevention of cancer.

13. The use according to claim 12, which comprises the step of generating a glucosinolate and/or a glucosinolate breakdown product.

14. A method for enhancing pest and/or disease resistance in a plant which comprises the step of expressing an enzyme according to claim 1 in the plant.

15. A method for synthesising a glycoside which comprises the step of using an enzyme according to claim 1 to catalyse a transglycosylation reaction.

16. A method for synthesising a sulphated carbohydrate which comprises the step of using an enzyme according to claim 1 to catalyse a sulphation reaction.

17. A method for making a model for the three-dimensional structure of aphid myrosinase by
(i) comparing the amino acid sequence of an enzyme according to claim 1, with the amino acid sequence of a related enzyme for which a crystal structure has been determined and
(ii) modelling the three-dimensional structure of aphid myrosinase on the crystal structure of the related enzyme.

18. A plant expressing an enzyme according to claim 1.

## Patentansprüche

1. Myrosinaseenzym von einem nicht-pflanzlichen Ursprung, das die in SEQ ID Nr. 1 gezeigte Aminosäuresequenz aufweist, oder ein Homologes davon mit wenigstens 75 % Sequenzidentität und welches Myrosinaseaktivität zeigt und zur Aktivierung keine Ascorbinsäure erfordert.

2. Nucleotidsequenz, welche ein Enzym gemäß Patentanspruch 1 codiert.

3. Nucleotidsequenz nach Anspruch 2, welche die in SEQ ID Nr. 2 gezeigte Nucleotidsequenz umfaßt oder
(i) ein Homologes davon mit wenigstens 75 % Sequenzidentität,
(ii) ein Fragment davon, bei dem eine oder mehrere Basen fehlen, welches aber noch die Fähigkeit beibehalten hat, ein Enzym gemäß Patentanspruch 1 zu codieren, oder
(iii) ein Derivat davon, welches eine chemische Modifikation der Seitenketten und/oder des Rückrats der Nucleotidsequenz aufweist, welches aber die Fähigkeit beibehalten hat, ein Enzym gemäß Patentanspruch 1 zu codieren.

4. Vektor, der die Nucleotidsequenz gemäß Patentanspruch 2 oder 3 enthält.

5. Wirtszelle, in welche die Nucleotidsequenz gemäß Patentanspruch 2 oder 3 aufgenommen wurde.

6. Organismus, in welchen die Nucleotidsequenz gemäß Patentanspruch 2 oder 3 aufgenommen wurde und welcher ein Pilz, eine Hefe, eine Pflanze oder ein prokaryontischer Organismus ist.

7. Organismus nach Patentanspruch 6, wobei der Organismus eine Pflanze ist.

8. Antikörper, welcher das Enzym von Patentanspruch 1 erkennt.

9. Verfahren zur Durchmusterung eines Mittels, das in der Lage ist, Myrosinaseaktivität und/oder-expression zu modulieren, welches die folgenden Stufen umfaßt:
(i) inkontaktbringen eines Mittels mit einem Enzym gemäß Patentanspruch 1 oder einer Nucleinsäure gemäß Patentanspruch 2 oder 3,
(ii) Messen der Aktivität und/oder Expression von Myrosinase, wobei ein Unterschied zwischen a) Myrosinaseaktivität/-expression in der Abwesenheit des Mittels und b) Myrosinaseaktivität/-expression in der Gegenwart des Mittels ein Anzeichen dafür ist, daß das Mittel in der Lage ist, Myrosinaseaktivität und/oder-expression zu modulieren.

10. Verfahren nach Patentanspruch 9, wobei das Mittel in der Lage ist, die Aktivität und/oder Expression der Myrosinase zu hemmen oder zu blockieren.

11. Verfahren nach Anspruch 10, wobei das Mittel in der Lage ist, die Aktivität und/oder Expression von Kohlblattlausmyrosinase zu hemmen oder zu blockieren.

12. Verwendung eines Enzyms gemäß Patentanspruch 1 oder einer Nucleotidsequenz gemäß Patenanspruch 2 oder 3 bei der Herstellung eines Medikaments für die Behandlung von oder Vorbeugung gegen Krebs.

13. Verwendung nach Patenanspruch 12, welche die Stufe der Erzeugung eines Glucosinolates und/oder eines Glucosinolatabbauproduktes umfaßt.

14. Verfahren zur Erhöhung der Widerstandsfähigkeit gegen Pflanzenschädlinge und/oder Krankheit in einer Pflanze, welches die Stufe des Exprimierens eines Enzyms gemäß Patentanspruch 1 in der Pflanze umfaßt.

15. Verfahren zum Synthetisieren eines Glycosides, welches die Stufe der Verwendung eines Enzyms gemäß Patentanspruch 1 zum Katalysieren einer Transglycosilierungsreaktion umfaßt.

16. Verfahren zum Synthetisieren eines sulfatierten Kohlenhydrates, welches die Stufe der Verwendung eines Enzyms gemäß Patentanspruch 1 zum Katalysieren einer Sulfatierungsreaktion umfaßt.

17. Verfahren zur Herstellung eines Modells für die dreidimensionale Struktur von Blattlausmyrosinase durch
(i) Vergleichen der Aminosäuresequenz eines Enzyms gemäß Patentanspruch 1 mit der Aminosäuresequenz eines verwandten Enzyms, für welches eine Kristallstruktur bestimmt wurde, und
(ii) Modellieren der dreidimensionalen Struktur von Blattlausmyrosinase anhand der Kristallstruktur des verwandten Enzyms.

18. Pflanze, die ein Enzym gemäß Patentanspruch 1 exprimiert.

## Revendications

1. Enzyme myrosinase provenant d'une source non végétale comprenant la séquence d'aminoacides montrée dans la SEQ ID N° 1, ou un homologue de celle-ci ayant au moins 75% d'identité de séquence, qui présente une activité myrosinase, et qui ne requiert pas d'acide ascorbique pour une activation.

2. Séquence de nucléotides qui code pour une enzyme selon la revendication 1.

3. Séquence de nucléotides selon la revendication 2, comprenant la séquence d'acide nucléique montrée dans la SEQ ID N° 2 ou
(i) un homologue de celle-ci ayant au moins 75% d'identité de séquence ;
(ii) un fragment de celle-ci qui a une ou plusieurs bases manquantes, mais qui conserve la capacité de coder pour une enzyme selon la revendication 1 ; ou
(iii) un dérivé de celle-ci qui a une modification chimique des chaînes latérales et/ou du squelette de la séquence de nucléotides mais qui conserve la capacité de coder pour une enzyme selon la revendication 1.

4. Vecteur comprenant la séquence de nucléotides selon la revendication 2 ou 3.

5. Cellule hôte dans laquelle a été incorporée la séquence de nucléotides selon la revendication 2 ou 3.

6. Organisme dans lequel a été incorporée la séquence de nucléotides selon la revendication 2 ou 3, et qui est un champignon, une levure, une plante ou un organisme procaryote.

7. Organisme selon la revendication 6, ledit organisme étant une plante.

8. Anticorps qui reconnaît l'enzyme selon la revendication 1.

9. procédé permettant de sélectionner un agent capable de moduler l'activité et/ou l'expression de la myrosinase, qui comprend les étapes suivantes :
(i) mise en contact d'un agent avec une enzyme selon la revendication 1 ou un acide nucléique selon la revendication 2 ou 3;
(ii) mesure de Inactivité et/ou de l'expression de la myrosinase
dans lequel une différence entre a) l'activité/l'expression de la myrosinase en l'absence d'agent, et b) l'activitél/l'expression de la myrosinase en présence d'agent montre que l'agent est capable de moduler l'activité et/ou l'expression de la myrosinase.

10. Procédé selon la revendication 9, dans lequel l'agent est capable d'inhiber ou de bloquer l'activité et/ou l'expression de la myrosinase.

11. procédé selon la revendication 10, dans lequel l'agent est capable d'inhiber ou de bloquer l'activité et/ou l'expression de la myrosinase du puceron du chou.

12. Utilisation d'une enzyme selon la revendication 1 ou d'une séquence de nucléotides selon la revendication 2 ou 3, dans la fabrication d'un médicament destiné au traitement ou à la prévention d'un cancer.

13. Utilisation selon la revendication 12, qui comprend l'étape de formation d'un glucosinolate et/ou d'un produit de dégradation de glucosinolate.

14. Procédé permettant d'aluélzorer la résistance à un insecte nuisible et/ou une maladie chez une plante qui comprend l'étape d'expression d'une enzyme selon la revendication 1 dans la plante.

15. Procédé de synthèse d'un glycoside, qui comprend l'étape d'utilisation d'une enzyme selon la revendication 1 pour catalyser une réaction de transglycosylation.

16. Procédé de synthèse d'un hydrate de carbone sulfaté, qui comprend l'étape d'utilisation d'une enzyme selon la revendication 1 pour catalyser une réaction de sulfatation.

17. Procédé permettant d'élaborer un modèle pour la structure tridimensionnelle d'une myrosinase de puceron par
(i) comparaison de la séquence d'aminoacides d'une enzyme selon la revendication 1 avec la séquence d'aminoacides d'une enzyme apparentée pour laquelle une structure cristalline a été déterminée et
(ii) modélisation de la structure tridimensionnelle de la myrosinase de puceron sur la structure cristalline de l'enzyme apparentée.

18. Plante exprimant une enzyme selon la revendication 1.


### SEQUENCE LISTINGS

### SEQUENCE LISTING

<110> Imperial College Innovations Limited
<120> ENZYME
<130> P009485WO LCH
<140> PCT/GB01/03670
   <141> 2001-08-16
<160> 25
<170> PatentIn version 3.0
<210> 1
   <211> 464
   <212> PRT
   <213> Brevicoryne brassicae
<400> 1
   Met Asp Tyr Lys Phe Pro Lys Asp Phe Met Phe Gly Thr Ser Thr Ala
   1               5                   10                  15
   Ser Tyr Gln Ile Glu Gly Gly Trp Asn Glu Asp Gly Lys Gly Glu Asn
               20                  25                  30
   Ile Trp Asp Arg Leu Val His Thr Ser Pro Glu Val Ile Lys Asp Gly
           35                  40                  45
   Thr Asn Gly Asp Ile Ala Cys Asp Ser Tyr His Lys Tyr Lys Glu Asp
       50                  55                  60
   Val Ala Ile Ile Lys Asp Leu Asn Leu Lys Phe Tyr Arg Phe Ser Ile
   65                  70                  75                  80
   Ser Trp Ala Arg Ile Ala Pro Ser Gly Val Met Asn Ser Leu Glu Pro
                   85                  90                  95
   Lys Gly Ile Ala Tyr Tyr Asn Asn Leu Ile Asn Glu Leu Ile Lys Asn
               100                 105                 110
   Asp Ile Ile Pro Leu Val Thr Met Tyr His Trp Asp Leu Pro Gln Tyr
           115                 120                 125
   Leu Gln Asp Leu Gly Gly Trp Val Asn Pro Ile Met Ser Asp Tyr Phe
       130                 135                 140
   Lys Glu Tyr Ala Arg Val Leu Phe Thr Tyr Phe Gly Asp Arg Val Lys
   145                 150                 155                 160
   Trp Trp Ile Thr Phe Asn Glu Pro Ile Ala Val Cys Lys Gly Tyr Ser
                   165                 170                 175
   Ile Lys Ala Tyr Ala Pro Asn Leu Asn Leu Lys Thr Thr Gly His Tyr
               180                 185                 190
   Leu Ala Gly His Thr Gln Leu Ile Ala His Gly Lys Ala Tyr Arg Leu
           195                 200                 205
   Tyr Glu Glu Met Phe Lys Pro Thr Gln Asn Gly Lys Ile Ser Ile Ser
       210                 215                 220
   Ile Ser Gly Val Phe Phe Met Pro Lys Asn Ala Glu Ser Asp Asp Asp
   225                 230                 235                 240
   Ile Glu Thr Ala Glu Arg Ala Asn Gln Phe Glu Arg Gly Trp Phe Gly
                   245                 250                 255
   His Pro Val Tyr Lys Gly Asp Tyr Pro Pro Ile Met Lys Lys Trp Val
               260                 265                 270
   Asp Gln Lys Ser Lys Glu Glu Gly Leu Pro Trp Ser Lys Leu Pro Lys
           275                 280                 285
   Phe Thr Lys Asp Glu Ile Lys Leu Leu Lys Gly Thr Ala Asp Phe Tyr
       290                 295                 300
   Ala Leu Asn His Tyr Ser Ser Arg Leu Val Thr Phe Gly Ser Asp Pro
   305                 310                 315                 320
   Asn Pro Asn Phe Asn Pro Asp Ala Ser Tyr Val Thr Ser Val Asp Glu
                   325                 330                 335
   Ala Trp Leu Lys Pro Asn Glu Thr Pro Tyr Ile Ile Pro Val Pro Glu
               340                 345                 350
   Gly Leu Arg Lys Leu Leu Ile Trp Leu Lys Asn Glu Tyr Gly Asn Pro
           355                 360                 365
   Gln Leu Leu Ile Thr Glu Asn Gly Tyr Gly Asp Asp Gly Gln Leu Asp
       370                 375                 380
   Asp Phe Glu Lys Ile Ser Tyr Leu Lys Asn Tyr Leu Asn Ala Thr Leu
   385                 390                 395                 400
   Gln Ala Met Tyr Glu Asp Lys Cys Asn Val Ile Gly Tyr Thr Val Trp
                   405                 410                 415
   Ser Leu Leu Asp Asn Phe Glu Trp Phe Tyr Gly Tyr Ser Ile His Phe
               420                 425                 430
   Gly Leu Val Lys Ile Asp Phe Asn Asp Pro Gln Arg Thr Arg Thr Lys
           435                 440                 445
   Arg Glu Ser Tyr Thr Tyr Phe Lys Asn Val Val Ser Thr Gly Lys Pro
       450                 455                 460
<210> 2
   <211> 2281
   <212> DNA
   <213> Brevicoryne Brassicae
<220>
   <221> misc_feature
   <222> (2223)..(2236)
   <223> n=any
<400> 2
   aatctcgcta gtttacgcta ttctagttaa actctgttca aatttatcgg tgaactttat      60
   aagttaaatg tattaatgta tttagacatt gttgaattat aacacgaata ttcaaacgct     120
   ttggttaatt atttcaaaaa ttcttccatc tcatcaaacg gtttggactc gcgacaatca     180
   ataccaagtt tctcattgaa ctaaactcga caattaatca atttaagtat tcaatatgga     240
   ttataaattt ccaaaggatt ttatgtttgg cacttcaact gcctcatatc aaattgaagg    300
   aggctggaat gaagacggaa aaggagaaaa tatttgggat cgtttggttc atactagtcc    360
   agaagtaata aaagatggga ctaatggaga tattgcctgt gattcctatc acaagtataa    420
   agaagatgta gcaattataa aagatttgaa tttgaagttt tatcgttttt caatatcatg    480
   ggctcgaata gcaccatctg gagtaatgaa ttcattagaa ccaaaaggaa tagcatacta    540
   taataattta atcaatgaac ttatcaagaa tgatattatt cctttagtta cgatgtatca    600
   ttgggactta ccacaatacc tacaggatct tggaggttgg gttaatccaa taatgtcaga    660
   ttattttaaa gaatatgcac gagtgttatt tacttacttc ggagacagag taaaatggtg    720
   gataacattt aatgaaccaa tagctgtttg taaaggttat tccattaaag cctatgctcc    780
   aaacttgaat ttaaagacca ccggacatta tttagcaggt catacacaac ttattgcaca    840
   tggaaaagca tataggttgt atgaagaaat gtttaaacct acacaaaatg gaaaaataag    900
   tatttcaatt agtggagtgt ttttcatgcc aaaaaatgct gaatcagatg atgatataga    960
   aactgctgaa agagctaacc aatttgagag aggatggttc ggtcatccag tgtacaaggg   1020
   agactatcca cctataatga aaaaatgggt tgatcaaaag agtaaagaag aaggtttacc   1080
   atggtccaaa ttacctaaat ttacaaaaga tgaaataaaa ttacttaaag gtactgctga   1140
   tttttatgct ctcaatcatt attcgtctcg tttggtgact tttggaagtg atccaaatcc   1200
   taattttaat cctgacgcat cttatgttac ttctgtagac gaagcatggt taaagccgaa   1260
   tgaaacaccg tatattatac cagtacccga aggtttaaga aaacttttga tatggttaaa   1320
   aaacgaatat ggcaatcccc aattgcttat tacagaaaat ggatatggag acgacggtca   1380
   attggatgat tttgaaaaaa ttagctacct aaagaactat ttaaatgcaa cattacaagc   1440
   gatgtatgaa gataaatgca atgtaatagg atataccgtg tggtcactct tggacaattt   1500
   tgaatggttt tatggttatt cgattcattt tggacttgtt aagatagatt ttaatgaccc   1560
   tcaaagaact cgtactaaaa gagaatcata cacatatttc aagaatgtgg tatcaactgg   1620
   caaaccataa tatttataaa caccttcgat tagttaatat tagaaaaacg cttttatccg   1680
   aattatgaaa aatgtaattt taattaaata acaataaaca tatacacata atatacataa   1740
   catttcacaa tcaactctca acgcgataac accgaactaa atctatcgac taacatctat   1800
   aaacaggtac taagctggcc agccgtgcac cgactacata atgagccatt tatacttgta   1860
   acggttacat cactcaccaa aacattcttt ctaaggacta cacaatcaac caatcagaac   1920
   atgacataca ggaaataaga gcagtcagag acttttgatc aaatcttaat tctctggact   1980
   atactaatca acccgatact tacgaacatg gataggggag gtcaggacaa agataatgag  2040
   acccttcaca tggccgtaac ccagaaatcc agcaaacagc atccagatcc gttcagaaac   2100
   aaaaacaaca cggcggtaca cccgatagtt tactagtcgg tacagcacgc ggattatacc   2160
   ctttattttc ttcaatataa cattattata tagctaaata actatgtatt gctttttttt 2220
   ttnaaataaa attttntgaa ccntcntttt aaaaaaaaaa aaaaaaaaaa aaaaaaaaaa 2280
   a 2281
<210> 3
   <211> 26
   <212> DNA
   <213> Artificial
<220>
   <223> PCR primer
<220>
   <221> modified_base
   <222> (3) .. (3)
   <223> n=i
<220>
   <221> misc_feature
   <222> (18)..(24)
   <223> n=any
<400> 3
   gcntaytaya ayaayytnat hccngc 26
<210> 4
   <211> 20
   <212> DNA
   <213> Artificial
<220>
   <223> PCR primer
<220>
   <221> n
   <222> (3)..(18)
   <223> n=any
<220>
   <221> r
   <222> (6)..(12)
   <223> r=g or a
<400> 4
   canggrtgnc craaccancc 20
<210> 5
   <211> 23
   <212> DNA
   <213> Artificial
<220>
   <223> PCR primer
<220>
   <221> modified_base
   <222> (6)..(9)
   <223> n=inosine
<220>
   <221> mise feature
   <222> (12)..(12)
   <223> n=any
<400> 5
   twygtnacny tnttycaytg ggc 23
<210> 6
   <211> 23
   <212> DNA
   <213> Artificial
<220>
   <223> PCR primer
<220>
   <221> modified_base
   <222> (3)..(6)
   <223> n=any
<220>
   <221> n
   <222> (9)..(20)
   <223> n=any
<400> 6
   gtnarnggnt ccatrwacca ncc 23
<210> 7
   <211> 15
   <212> PRT
   <213> Brevicoryne Brassicae
<400> 7
   Leu Val Thr Phe Gly Ser Asp Pro Asn Asn Asn Phe Asn Pro Asp
   1 5 10 15
<210> 8
   <211> 14
   <212> PRT
   <213> Brevicoryne Brassicae
<400> 8
   Gly Ile Ala Tyr Tyr Asn Asn Leu Ile Pro Glu Leu Ile Lys
   1 5 10
<210> 9
   <211> 9
   <212> PRT
   <213> Brevicoryne Brassicae
<400> 9
   Gly Trp Phe Gly His Pro Val Tyr Lys
   1 5
<210> 10
   <211> 10
   <212> PRT
   <213> Brevicoryne Brassicae
<400> 10
   Thr Thr Gly His Tyr Leu Ala Gly His Thr
   1 5 10
<210> 11
   <211> 5
   <212> PRT
   <213> Brevicoryne Brassicae
<400> 11
   Ile Ser Tyr Leu Lys
   1 5
<210> 12
   <211> 24
   <212> DNA
   <213> Artificial
<220>
   <223> 5' PCR primer in Example 9
<400> 12
   attccatatg gattataaat ttcc 24
<210> 13
   <211> 30
   <212> DNA
   <213> Artificial
<220>
   <223> 3' PCR primer in Example 9
<400> 13
   tataactcga gtggtttgcc agttgatacc 30
<210> 14
   <211> 27
   <212> DNA
   <213> Artificial
<220>
   <223> 5' PCR primer in Example 9
<400> 14
   gtagctcgag tggattataa atttcca 27
<210> 15
   <211> 50
   <212> DNA
   <213> Artificial
<220>
   <223> 3' PCR primer in Example 9
<400> 15
   tatggatccc ttaatggtga tgatggtgat gtggtttgcc agttgatacc 50
<210> 16
   <211> 30
   <212> PRT
   <213> Caldicellulosiruptor saccharolyticus
<400> 16
   Phe Pro Lys Gly Phe Leu Trp Gly Ala Ala Thr Ala Ser Tyr Gln Ile
   1               5                   10                  15
   Glu Gly Ala Trp Asn Glu Asp Gly Lys Gly Glu Ser Ile Trp
               20                  25                  30
<210> 17
   <211> 30
   <212> PRT
   <213> Brevicoryne Brassicae
<400> 17
   Phe Pro Lys Asp Phe Met Phe Gly Thr Ser Thr Ala Ser Tyr Gln Ile
   1               5                   10                  15
   Glu Gly Gly Trp Asn Glu Asp Gly Lys Gly Glu Asn Ile Trp
               20                  25                  30
<210> 18
   <211> 34
   <212> PRT
   <213> Trifolium repens
<400> 18
   Asp Gln Tyr His Arg Tyr Lys Glu Asp Val Gly Ile Met Lys Asp Gln
   1               5                   10                  15
   Asn Met Asp Ser Tyr Arg Phe Ser Ile Ser Trp Pro Arg Ile Leu Pro
               20                  25                  30
   Lys Gly
<210> 19
   <211> 34
   <212> PRT
   <213> Brevicoryne Brassicae
<400> 19
   Asp Ser Tyr His Lys Tyr Lys Glu Asp Val Ala Ile Ile Lys Asp Leu
   1               5                   10                  15
   Asn Leu Lys Phe Tyr Arg Tyr Ser Ile Ser Trp Ala Arg Ile Ala Pro
               20                  25                  30
   Ser Gly
<210> 20
   <211> 35
   <212> PRT
   <213> Trifolium repens
<400> 20
   Asn His Glu Gly Ile Lys Tyr Tyr Asn Asn Leu Ile Asn Glu Leu Leu
   1               5                   10                  15
   Ala Asn Gly Ile Gln Pro Phe Val Thr Leu Phe His Trp Asp Leu Pro
               20                  25                  30
   Gln Val Leu
           35
<210> 21
   <211> 35
   <212> PRT
   <213> Brevicoryne Brassicae
<400> 21
   Glu Pro Lys Gly Ile Ala Tyr Tyr Asn Asn Leu Ile Asn Glu Leu Ile
   1 5 10 15
   Lys Asn Asp Ile Ile Pro Leu Val Thr Met Tyr His Trp Asp Leu Pro
   20 25 30
   Gln Tyr Leu
   35
<210> 22
   <211> 12
   <212> PRT
   <213> Microspora bispora
<400> 22
   Leu Ile Ile Thr Glu Asn Gly Ala Ala Phe Asp Asp
   1 5 10
<210> 23
   <211> 12
   <212> PRT
   <213> Brevicoryne Brassicae
<400> 23
   Leu Leu Ile Thr Glu Asn Gly Tyr Gly Asp Asp Gly
   1 5 10
<210> 24
   <211> 28
   <212> PRT
   <213> clostridium thermocellum
<400> 24
   Asp Gly Val Asn Leu Lys Ala Tyr Tyr Leu Trp Ser Leu Leu Asp Asn
   1 5 10 15
   Phe Glu Trp Ala Tyr Gly Tyr Asn Lys Arg Phe Gly
   20 25
<210> 25
   <211> 28
   <212> PRT
   <213> Brevicoryne Brassicae
<400> 25
   Asp Lys Cys Asn Val Ile Gly Tyr Thr Val Trp Ser Leu Leu Asp Asn
   1 5 10 15
   Phe Glu Trp Phe Tyr Gly Tyr Ser Ile His Phe Gly
   20 25